# EUROPEAN PATENT APPLICATION

(11) **EP 4 796 115 A2**
(43) Date of publication of application: **26.08.2026**
(21) Application number: 26176653.9
(22) Date of filing: 18.09.2019
(51) Int. Cl.: A61K 31/535

(54) **INTEGRASE INHIBITORS FOR THE PREVENTION OF HIV**

(30) Priority: 19.09.2018 US 201862733536 P
(62) Document number(s) of the earlier application(s) in accordance with Art. 76 EPC: 19780084.0 / 3 852 761
(71) Applicant: Gilead Sciences, Inc., Foster City, CA 94404 (US)
(72) Inventor: BEKERMAN, Elena, Foster City, 94404 (US); CALLEBAUT, Christian, Foster City, 94404 (US); MCCALLISTER, Scott, San Francisco, 94122 (US)
(74) Representative: Carpmaels & Ransford LLP

(57) **Abstract**

The present invention provides methods of preventing HIV in a subject, comprising administering to the subject a therapeutically effective amount of bictegravir, or a pharmaceutically acceptable salt thereof, optionally in combination with one or more additional therapeutic agents. Methods of reducing the risk of acquiring HIV (*e.g.,* HIV-1 and/or HIV-2) are also provided.

## Description

### CROSS REFERENCE TO RELATED APPLICATIONS

This Application claims the benefit of U.S. Provisional Application 62/733,536, filed on September 19, 2018, the entire content of which is hereby incorporated by reference in its entirety.

### FIELD

The present invention provides methods of preventing HIV in a subject comprising administering to the subject a therapeutically effective amount of an HIV integrase strand transfer inhibitor (*e*.*g*., elvitegravir or bictegravir), or a pharmaceutically acceptable salt thereof, optionally in combination with one or more other therapeutic agents.

### BACKGROUND

The HIV/AIDS pandemic has claimed the lives of millions of people, and millions more are currently infected. Antiretroviral therapy has turned HIV infection into a chronic, manageable disease; however, no cure yet exists for HIV. Reduction in the number of new HIV infections is a global goal. To this end, prevention regimens relating to both pre-exposure prophylaxis (PrEP) and post-exposure prophylaxis (PEP) are being explored. Truvada (emtricitabine-tenofovir) is currently the only medication approved for PrEP. Accordingly, new and effective means for preventing HIV infection are needed and the methods described herein are developed to help meet this need.

### SUMMARY

The present invention provides a method of preventing an HIV infection in a subject, or a method of reducing the risk of acquiring HIV, comprising administering to the subject a therapeutically effective amount of an HIV integrase strand transfer inhibitor (*e*.*g*., elvitegravir or bictegravir), or a pharmaceutically acceptable salt thereof, optionally in combination with one or more other therapeutic agents.

### BRIEF DESCRIPTION OF THE DRAWINGS

FIG. 1 shows a representative scheme illustrating the event driven study design of Example 5.
FIG. 2 shows a PrEP/PEP study design schematic further described in Example 6.
FIG. 3 shows survival in the PrEP/PEP study animals as a percent of SHIV-negative animals per group following eight cycles of low-dose rectal challenge (see Example 6).
FIG. 4 shows a PEP study design schematic further described in Example 6.
FIG. 5 shows survival in the PEP study animals as a percent of SHIV-negative animals per group following five rectal challenge (see Example 6).

### DETAILED DESCRIPTION

The present invention relates to a method of preventing an HIV infection (*e*.*g*., HIV-1 and/or HIV-2) in a subject (*e*.*g*., a human) by administering to the subject a therapeutically effective amount of an HIV integrase strand transfer inhibitor (*e*.*g*., elvitegravir or bictegravir), or a pharmaceutically acceptable salt thereof. In some embodiments, the HIV integrase strand transfer inhibitor (*e*.*g*., elvitegravir or bictegravir), or a pharmaceutically acceptable salt thereof, is administered as a monotherapy (*i*.*e*., in the absence of an additional therapeutic agent). In some embodiments, the HIV integrase strand transfer inhibitor (*e*.*g*., elvitegravir or bictegravir), or a pharmaceutically acceptable salt thereof, is administered in combination with one or more other therapeutic agents, such as anti-HIV agents. In some embodiments, the HIV integrase strand transfer inhibitor (*e*.*g*., elvitegravir or bictegravir) is administered as bictegravir sodium.

In some embodiments, the subject may have or be at risk of having the infection. In some embodiments, the subject has been identified as an individual who is at risk of sexual transmission of HIV. In some embodiments, the individual has been identified as a man, transgender man, transgender woman, or woman who has sex with men, a heterosexual men, a heterosexual woman, and or a sex worker. In some embodiments, the individual has been identified as:
- having anal sex with at least two different sexual partners and no consistent condom use over the last 6 months; and/or
- having history of sexually transmitted diseases (STDs) during the last 12 months (*e*.*g*., syphilis, gonorrhea, chlamydiae, HBV or HCV infection); and/or
- using psycho-actives drugs during sexual intercourses (*e*.*g*., cocaine, gammahydroxybutyric acid (GHB), methylenedioxymethamphetamine (MDMA), mephedrone); and/or
- having sexual intercourse with one or more partners originating from a region with high prevalence of HIV infection (> 1%) (*e*.*g*., South America, Sub-Saharan Africa, South-East Asia, Eastern Europe, French Guyana) and no consistent condom use; and/or
- a sex worker; and/or
- having a sexual partner who is an intravenous drug user sharing injection material; and/or
- having an HIV-infected sexual partner with a detectable plasma viral load (*e*.*g*., >50 copies (cp)/milliliter (mL)).

In some embodiments, the subject is HIV-negative. In some embodiments, the HIV is HIV-1 and/or HIV-2.

As used herein, the terms "prevention" or "preventing" refers to the administration of a compound, composition, or pharmaceutically salt according to the present disclosure pre- or post-exposure of the human to the virus but before the appearance of symptoms of the disease, and/or prior to the detection of the virus in the blood. The terms also refer to prevention of the appearance of symptoms of the disease and/or to prevent the virus from reaching detectible levels in the blood. The term includes both pre-exposure prophylaxis (PrEP), as well as post-exposure prophylaxis (PEP) and event driven or "on demand" prophylaxis. The term also refers to prevention of perinatal transmission of HIV from mother to baby, by administration to the mother before giving birth and to the child within the first days of life. The term also refers to prevention of transmission of HIV through blood transfusion.

As used herein, "bictegravir" or "BIC" each refer to the integrase inhibitor drug compound (2R,5S,13aR)-8-hydroxy-7,9-dioxo-N-(2,4,6-trifluorobenzyl)-2,3,4,5,7,9,13,13a-octahydro-2,5-methanopyrido[1',2':4,5]pyrazino[2,1-b][1,3]oxazepine-10-carboxamide (IUPAC name) represented by the below structure (Formula (I)). Bictegravir is described in U.S. Patent No.: 9,216,996, the disclosure of which is incorporated herein by reference in its entirety.

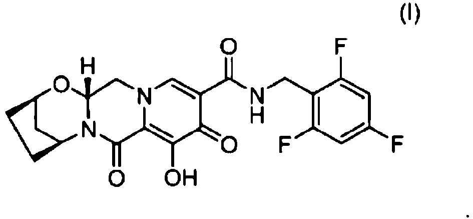

The term bictegravir further includes its pharmaceutically acceptable salts including, for example, its mono sodium salt.

As used herein, "elvitegravir" or "EVG" each refer to the integrase inhibitor drug compound 6-(3-chloro-2-fluorobenzyl)-1-[(2S)-1-hydroxy-3-methylbutan-2-yl]-7-methoxy-4-oxo-1,4-dihydroquinoline-3-carboxylic acid represented by the below structure (Formula (II)). Elvitegravir is described in U.S. Patent No.: 9,216,996, the disclosure of which is incorporated herein by reference in its entirety.

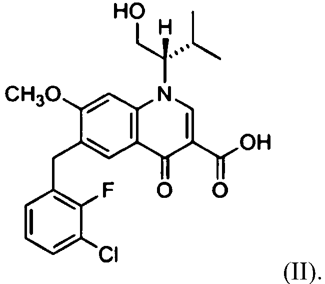

The term elvitegravir further includes its pharmaceutically acceptable salts including, for example, its mono sodium salt.

In the absence of a specific reference to a particular pharmaceutically acceptable salt and/or solvate of the above provided compound of Formula (I) or Formula (II), any dosages, whether expressed in milligrams or as % by weight, should be understood as referring to the amount of the free acid, i.e., the compound of Formula (I) or Formula (II). For example a reference to "50 mg" of bictegravir, or a pharmaceutically acceptable salt thereof, refers to an amount of the compound of Formula (I), or a pharmaceutically acceptable salt thereof, which provides the same amount of the compound of Formula (I) as 50 mg of the compound of Formula (I) free acid. In some embodiments, a dosage referring to 50 mg of bictegravir contains about 52 mg of bictegravir monosodium salt.

As used herein, "tenofovir alafenamide" or "TAF" each refer to the nucleoside analog reverse transcriptase inhibitor drug compound {9-[(R)-2-[[(S)-[[(S)-1-(isopropoxycarbonyl)ethyl] amino]phenoxyphosphinyl]-methoxy]propyl]adenine} having the structure shown below.

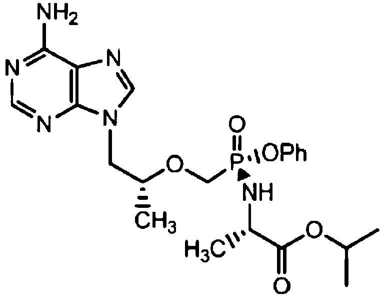

TAF may be associated with fumarate, such as monofumarate and hemifumarate salts or co-crystal (co-formers). See, *e.g.*, U.S. Patent Nos. 7,390,791, 7,803,788, and 8,754,065, each of which is hereby incorporated by reference in its entirety. It is understood that reference to "TAF" may be inclusive of a co-formers, such as fumarate. TAF is the active pharmaceutical ingredient in Vemlidy^{®} and is a component of the tablets Bictarvy^{®}, Genvoya^{®}, Descovy^{®}, Odefsey^{®}, and Symtuza^{®}.

In the absence of specific reference to a particular pharmaceutically acceptable salt and/or solvate of tenofovir alafenamide, any dosages, whether expressed in milligrams or as a % by weight, should be understood as referring to the amount of tenofovir alafenamide free base. For example reference to 25 mg of tenofovir alafenamide, or a pharmaceutically acceptable salt and/or solvate thereof, refers to an amount of tenofovir alafenamide, or a pharmaceutically acceptable salt and/or solvate thereof, which provides the same amount of tenofovir alafenamide as 25 mg of tenofovir alafenamide free base. In some embodiments, a dosage referring to 25 mg of tenofovir alafenamide contains about 28 mg of tenofovir alafenamide hemifumarate.

As used herein, "tenofovir disoproxil" or "TD" each refer to the compound 9-[(R)-2-[[bis[[(isopropoxycarbonyl)oxy] methoxy]phosphinyl]methoxy]propyl]adenine. TD, a prodrug of tenofovir, may be associated with fumarate, such as monofumarate. See *e*.*g*., U.S. Patent Nos. 5,922,695, 5,935,946, and 5,977,089, each of which is hereby incorporated by reference in its entirety. Tenofovir disoproxil fumarate is referred to as "TDF" and is the active pharmaceutical ingredient in Viread^{®}.

In the absence of specific reference to a particular pharmaceutically acceptable salt and/or solvate of tenofovir disoproxil, any dosages, whether expressed in milligrams or as a % by weight, should be taken as referring to the amount of tenofovir disoproxil free base. For example, reference to 245 mg tenofovir disoproxil, or a pharmaceutically acceptable salt and/or solvate thereof, refers to an amount of tenofovir disoproxil or a pharmaceutically acceptable salt and/or solvate thereof which provides the same amount of tenofovir disoproxil as 245 mg of tenofovir disoproxil free base. In some embodiments, a dosage referring to 245 mg of tenofovir disoproxil contains about 300 mg of tenofovir disoproxil fumarate.

As used herein, "emtricitabine" or "FTC" each refer to the compound 4-amino-5-fluoro-1-[(2R,5S)-2-(hydroxymethyl)-1,3-oxathiolan-5-yl]-1,2-dihydropyrimidin-2-one having the below structure.

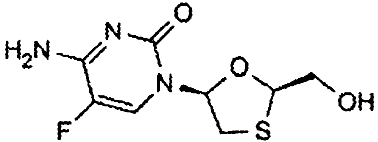

Emtricitabine can be present in dosage forms as a free base or as a pharmaceutically acceptable salt. Additionally, emtricitabine can be present in dosage forms in solvated or unsolvated forms. Typically, emtricitabine is present as a free base.

The present disclosure further provides that for any of the embodiments provided herein, emtricitabine, or a pharmaceutically acceptable salt thereof, can be replaced by lamivudine (*i*.*e*., 3TC), or a pharmaceutically acceptable salt thereof, in any appropriate dosage (*e*.*g*., 10 mg/day to 300 mg/day; 100 mg/day to 200 mg/day; 150 mg/day, and the like), or combination with other additional therapeutic agents, including the compounds of Formula (I) and Formula (II), or pharmaceutically acceptable salts thereof, as described herein.

In the absence of specific reference to a particular pharmaceutically acceptable salt and/or solvate of emtricitabine, any dosages, whether expressed in milligrams or as a % by weight, should be taken as referring to the amount of emtricitabine free base. For example, reference to 200 mg emtricitabine or a pharmaceutically acceptable salt and/or solvate thereof refers to an amount of emtricitabine or a pharmaceutically acceptable salt and/or solvate thereof which provides the same amount of emtricitabine as 200 mg of emtricitabine free base.

As used herein, "cobicistat" or "cobi" each refer to the compound 2,7,10,12-tetraazatridecanoic acid, 12-methyl-13-[2-(1-methylethyl)-4-thiazolyl]-9-[2-(4-morpholinyl)ethyl]-8,11-dioxo-3,6-bis(phenylmethyl)-, 5-thiazolylmethyl ester, (3R,6R,9S)-having the below structure.

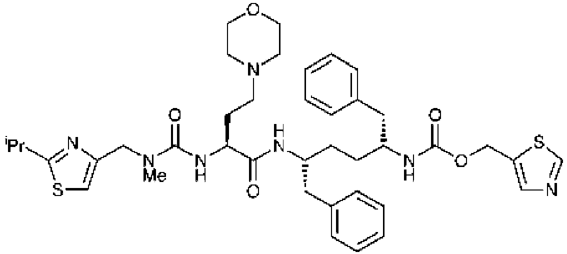

Cobicistat can be present in dosage forms as a free base or as a pharmaceutically acceptable salt. Additionally, cobicistat can be present in dosage forms in solvated or unsolvated forms. Typically, cobicistat is present as a free base. In certain embodiments, cobicistat is present in pharmaceutical compositions in combination with elvitegravir.

In the absence of specific reference to a particular pharmaceutically acceptable salt and/or solvate of cobicistat, any dosages, whether expressed in milligrams or as a % by weight, should be taken as referring to the amount of cobicistat free base. For example, reference to 200 mg cobicistat or a pharmaceutically acceptable salt and/or solvate thereof refers to an amount of cobicistat or a pharmaceutically acceptable salt and/or solvate thereof which provides the same amount of cobicistat as 200 mg of cobicistat free base.

In some embodiments, bictegravir, or a pharmaceutically acceptable salt thereof, is administered to the subject in a dosage of from about 1 mg/day to about 200 mg/day, for example, about 1 mg/day, about 5 mg/day, about 10 mg/day, about 25 mg/day, about 50 mg/day, about 75 mg/day, about 100 mg/day, about 125 mg/day, about 150 mg/day, about 175 mg/day, or about 200 mg/day. In some embodiments, bictegravir, or a pharmaceutically acceptable salt thereof, is administered to the subject in a dosage of from about 10 mg/day to about 100 mg/day. In some embodiments, bictegravir, or a pharmaceutically acceptable salt thereof, is administered in a dosage of from about 50 mg/day to about 100 mg/day. In some embodiments, bictegravir, or a pharmaceutically acceptable salt thereof, is administered in a dosage of about 75 mg/day. In some embodiments, bictegravir, or a pharmaceutically acceptable salt thereof, is administered in a dosage of from about 45 mg/day to about 55 mg/day. In some embodiments, bictegravir, or a pharmaceutically acceptable salt thereof, is administered in a dosage of about 50 mg/day. In some embodiments, the bictegravir is a pharmaceutically acceptable salt of bictegravir. In some embodiments, the bictegravir is bictegravir sodium salt. In some embodiments, the bictegravir is administered as the sodium salt in a dosage of about 52 mg/day (*e*.*g*., 52.5 mg/day).

In some embodiments, bictegravir, or a pharmaceutically acceptable salt thereof, is administered to the subject in a dosage of from about 10 mg/day to about 200 mg/day. In some embodiments, bictegravir, or a pharmaceutically acceptable salt thereof, is administered to the subject in a dosage of from about 50 mg/day to about 200 mg/day. In some embodiments, bictegravir, or a pharmaceutically acceptable salt thereof, is administered to the subject in a dosage of from about 50 mg/day to about 150 mg/day. In some embodiments, bictegravir, or a pharmaceutically acceptable salt thereof, is administered in a dosage of about 100 mg/day. In some embodiments, the bictegravir is administered as the sodium salt in a dosage of about 104 mg/day (*e*.*g*., 105 mg/day).

In some embodiments, elvitegravir, or a pharmaceutically acceptable salt thereof, is administered to the subject in a dosage of from about 1 mg/day to about 200 mg/day, for example, about 1 mg/day, about 5 mg/day, about 10 mg/day, about 25 mg/day, about 50 mg/day, about 75 mg/day, about 100 mg/day, about 125 mg/day, about 150 mg/day, about 175 mg/day, or about 200 mg/day. In some embodiments, elvitegravir, or a pharmaceutically acceptable salt thereof, is administered to the subject in a dosage of from about 100 mg/day to about 200 mg/day. In some embodiments, elvitegravir, or a pharmaceutically acceptable salt thereof, is administered in a dosage of from about 125 mg/day to about 175 mg/day. In some embodiments, elvitegravir, or a pharmaceutically acceptable salt thereof, is administered in a dosage of from about 150 mg/day to about 160 mg/day. In some embodiments, elvitegravir, or a pharmaceutically acceptable salt thereof, is administered in a dosage of about 150 mg/day. In some embodiments, the elvitegravir is a pharmaceutically acceptable salt of elvitegravir. In some embodiments, the elvitegravir is elvitegravir sodium salt. In some embodiments, the elvitegravir is administered as the sodium salt in a dosage of about 157 mg/day.

In some embodiments, cobicistat, or a pharmaceutically acceptable salt thereof, is administered to the subject in a dosage of from about 1 mg/day to about 200 mg/day, for example, about 1 mg/day, about 5 mg/day, about 10 mg/day, about 25 mg/day, about 50 mg/day, about 75 mg/day, about 100 mg/day, about 125 mg/day, about 150 mg/day, about 175 mg/day, or about 200 mg/day. In some embodiments, cobicistat, or a pharmaceutically acceptable salt thereof, is administered to the subject in a dosage of from about 100 mg/day to about 200 mg/day. In some embodiments, cobicistat, or a pharmaceutically acceptable salt thereof, is administered in a dosage of from about 125 mg/day to about 175 mg/day. In some embodiments, cobicistat, or a pharmaceutically acceptable salt thereof, is administered in a dosage of from about 150 mg/day to about 160 mg/day. In some embodiments, cobicistat, or a pharmaceutically acceptable salt thereof, is administered in a dosage of about 150 mg/day.

In some embodiments, the compound of Formula (I) or Formula (II) provided herein, or a pharmaceutically acceptable salt thereof, is administered daily. In certain embodiments, the methods disclosed herein involve repeated administrations at intervals less than once daily. For example, in certain embodiments, the methods disclosed herein involve administration of the compound of Formula (I) or Formula (II), or a pharmaceutically acceptable salt thereof, every other day, five times per week, four times per week, three times per week, two times per week, or one time per week.

In some embodiments, the methods disclosed herein comprise event driven administration of the compound of Formula (I) or Formula (II), or a pharmaceutically acceptable salt thereof, to the subject.

As used herein, the terms "event driven" or "event driven administration" refer to administration of the compound of Formula (I) or Formula (II), or a pharmaceutically acceptable salt thereof, (1) prior to an event (*e*.*g*., 2 hours, 1 day, 2 days, 5 day, or 7 or more days prior to the event) that would expose the individual to HIV (or that would otherwise increase the individual's risk of acquiring HIV); and/or (2) during an event (or more than one recurring event) that would expose the individual to HIV (or that would otherwise increase the individual's risk of acquiring HIV); and/or (3) after an event (or after the final event in a series of recurring events) that would expose the individual to HIV (or that would otherwise increase the individual's risk of acquiring HIV). In some embodiments, the event driven administration is performed pre-exposure of the subject to the HIV. In some embodiments, the event driven administration is performed post-exposure of the subject to the HIV. In some embodiments, the event driven administration is performed pre-exposure of the subject to the HIV and post-exposure of the subject to the HIV.

In certain embodiments, the methods disclosed herein involve administration prior to and/or after an event that would expose the individual to HIV or that would otherwise increase the individual's risk of acquiring HIV, *e*.*g*., as pre-exposure prophylaxis (PrEP) and/or as post-exposure prophylaxis (PEP). Examples of events that could increase an individual's risk of acquiring HIV include, without limitation, no condom use during anal intercourse with an HIV positive partner or a partner of unknown HIV status; anal intercourse with more than 3 sex partners; exchange of money, gifts, shelter or drugs for anal sex; sex with male partner and diagnosis of sexually transmitted infection; and no consistent use of condoms with sex partner known to be HIV positive. In some embodiments, the methods disclosed herein comprise pre-exposure prophylaxis (PrEP). In some embodiments, methods disclosed herein comprise post-exposure prophylaxis (PEP).

In some embodiments, the compound of Formula (I) or Formula (II), or a pharmaceutically acceptable salt thereof, is administered before exposure of the subject to the HIV.

In some embodiments, the compound of Formula (I) or Formula (II), or a pharmaceutically acceptable salt thereof, is administered before and after exposure of the subject to the HIV.

In some embodiments, the compound of Formula (I) or Formula (II), or a pharmaceutically acceptable salt thereof, is administered after exposure of the subject to the HIV.

An example of event driven dosing regimen includes administration of the compound of Formula (I) or Formula (II), or a pharmaceutically acceptable salt thereof, within 24 to 2 hours prior to HIV exposure (*e*.*g*., first sexual activity with sex partner known to be HIV positive, including sexual intercourse), followed by administration of the compound of Formula (I) or Formula (II), or a pharmaceutically acceptable salt, every 24 hours during the period of exposure (*e*.*g*., sexual activity with sex partner known to be HIV positive), followed by a further administration of the compound of Formula (I) or Formula (II), or a pharmaceutically acceptable salt thereof, after the last exposure (*e*.*g*., sexual activity with sex partner known to be HIV positive), and one last administration of the compound of Formula (I) or Formula (II), or a pharmaceutically acceptable salt thereof, 24 hours later.

A further example of an event driven dosing regimen includes administration of the compound of Formula (I) or Formula (II), or a pharmaceutically acceptable salt thereof, within 24 hours before HIV exposure (*e*.*g*., sexual activity with sex partner known to be HIV positive), then daily administration during the period of exposure (*e*.*g*., sexual activity with sex partner known to be HIV positive, including the last sexual intercourse), followed by a last administration approximately 24 hours later after the last exposure (which may be an increased dose, such as a double dose).

An example of continuous PrEP, includes for example, administration of the compound of Formula (I) or Formula (II), or a pharmaceutically acceptable salt thereof, every 24 hours, at least 7 days before the exposure (*e*.*g*., sexual activity with sex partner known to be HIV positive). Then when PrEP is to be discontinued, administration 24 hours after the last exposure (*e*.*g*., activity with sex partner known to be HIV positive, such as the last sexual intercourse, optionally as a double dose). If PrEP is to be resumed, administration can be every 24 hours, started at least 7 days before the exposure (*e*.*g*., activity with sex partner known to be HIV positive, such as the resumption of sexual intercourse) or administration (*e*.*g*., of a double dose) at least 2 hours before the exposure (*e*.*g*., activity with sex partner known to be HIV positive, such as resumption of sexual intercourse) and then administration every 24 hours.

In certain embodiments, *e*.*g*., when administered as PrEP, the compound of Formula (I) or Formula (II), or a pharmaceutically acceptable salt thereof, is administered 1 hour to 10 days, 1 hour to 7 days, 1 hour to 5 days, 1 to 72 hours, 1 to 48 hours, 1 to 24 hours, or 12 to 12 hours prior to an event that would increase the individual's risk of acquiring HIV (*e*.*g*., prior to sex or other exposure to the HIV virus). In some embodiments, the compound of Formula (I) or Formula (II), or a pharmaceutically acceptable salt thereof, is administered within 10 days, 7 days, 5 days, 72 hours, 60 hours, 48 hours, 24 hours, 12 hours, 9 hours, 6 hours, 4 hours, 3 hours, 2 hours, or 1 hour prior to an event that would increase the individual's risk of acquiring HIV (*e*.*g*., prior to sex or other exposure to the HIV virus). In certain embodiments, when the compound of Formula (I) or Formula (II), or a pharmaceutically acceptable salt thereof, is administered prior to an event (*e*.*g*., administered prior to the event) that would increase the individual's risk of acquiring HIV, it is administered daily prior to the event (*e*.*g*., sexual activity). In certain embodiments, when the compound of Formula (I) or Formula (II), or a pharmaceutically acceptable salt thereof, is administered prior to an event that would increase the individual's risk of acquiring HIV, it is administered one to three times prior to the event.

In some embodiments, the compound of Formula (I) or Formula (II), or a pharmaceutically acceptable salt thereof, is administered from about 72 hours to about 1 hour before exposure of the subject to the HIV. In some embodiments, the compound of Formula (I) or Formula (II), or a pharmaceutically acceptable salt thereof, is administered from about 24 hours to about 1 hour before exposure of the subject to the HIV. In some embodiments, the compound of Formula (I) or Formula (II), or a pharmaceutically acceptable salt thereof, is administered from about 72 hours to about 24 hours before exposure of the subject to the HIV.

In certain embodiments where the compound of Formula (I) or Formula (II), or a pharmaceutically acceptable salt thereof, is administered before exposure of the subject to the HIV, the methods disclosed herein further comprise administering one or more additional doses of the compound of Formula (I) or Formula (II), or a pharmaceutically acceptable salt thereof, during, and/or after exposure of the subject to the HIV.

In some embodiments, *e*.*g*., when administered as part of a PrEP regimen or as part of a PEP regimen, the compound of Formula (I) or Formula (II), or a pharmaceutically acceptable salt thereof, is administered during the time of HIV-exposure. In certain embodiments wherein the compound of Formula (I) or Formula (II) is administered before exposure, the compound of Formula (I) or Formula (II), or a pharmaceutically acceptable salt thereof, is administered daily (*e*.*g*., as a single dose) during the time of HIV-exposure (*e*.*g*., during the time period of sexual activity with sex partner known to be HIV positive). In some embodiments, the compound of Formula (I) or Formula (II), or a pharmaceutically acceptable salt thereof, is administered daily (*e*.*g*., for 1 to 7 days) after final exposure to the HIV (*e*.*g*., after a period of sexual activity with sex partner known to be HIV positive). In some embodiments, the administration is continued for 1 or 2 days after final exposure to HIV.

In some embodiments, the final dose of the compound of Formula (I) or Formula (II), or a pharmaceutically acceptable salt thereof, is increased, *e*.*g*., as a double dose, as a triple dose, and the like. In some embodiments, the increased dose of the compound of Formula (I) or Formula (II), or a pharmaceutically acceptable salt thereof, is a double dose. In some embodiments, the increased dose of the compound of Formula (I) or Formula (II), or a pharmaceutically acceptable salt thereof, is administered daily (*e*.*g*., for 1 to 7 days) after final exposure to the HIV (*e*.*g*., after a period of sexual activity with sex partner known to be HIV positive). In some embodiments, the increased dose of the compound of Formula (I) or Formula (II), or a pharmaceutically acceptable salt thereof, is administered for 1 or 2 day after final exposure to the HIV (*e*.*g*., after a period of sexual activity with sex partner known to be HIV positive).

In some embodiments, the compound of Formula (I) or Formula (II), or a pharmaceutically acceptable salt thereof, is administered as a single dose from about 2 to about 24 hours before exposure of the subject to the HIV. In some embodiments, the compound of Formula (I) or Formula (II), or a pharmaceutically acceptable salt thereof, is administered as a single dose about 24 hours after exposure of the subject to the HIV. In some embodiments, the compound of Formula (I) or Formula (II), or a pharmaceutically acceptable salt thereof, is administered as a single dose about 48 hours after exposure of the subject to the HIV.

Additional examples of PrEP and/or PEP can be found, for example, at the clinical trial summary titled "On Demand Antiretroviral Pre-exposure Prophylaxis for HIV Infection in Men Who Have Sex With Men" (Clinical Trial # NCT01473472); the clinical trial summary titled "Prevention of HIV in Île-de-France" (Clinical Trials # NCT03113123), and at Molina et al, N. Engl. J. Med. 2015, 353:2237-2246, the disclosure of each of which is incorporated herein by reference in its entirety.

In some embodiments, the compound of Formula (I) or Formula (II), or a pharmaceutically acceptable salt thereof, is administered:
i) beginning at least 7 days before exposure of the subject to the HIV (*e*.*g*., as a daily dose);
ii) during exposure of the subject to the HIV (*e*.*g*., as a daily dose); and
iii) for about 1 to 7 days after final exposure of the subject to the HIV (*e*.*g*., as a daily dose), wherein each of the doses after final exposure to the HIV is optionally a double dose.

In some embodiments, the compound of Formula (I) or Formula (II), or a pharmaceutically acceptable salt thereof, is administered:
i) beginning at least 48 hours before exposure of the subject to the HIV (*e*.*g*., as a daily dose);
ii) during exposure of the subject to the HIV (*e*.*g*., as a daily dose); and
iii) for about 1 to 2 days after final exposure of the subject to the HIV (*e*.*g*., as a daily dose), wherein each of the doses after final exposure to the HIV is optionally a double dose.

In some embodiments, the compound of Formula (I) or Formula (II), or a pharmaceutically acceptable salt thereof, is administered:
i) as a single or double dose from about 2 to about 24 hours before exposure of the subject to the HIV;
ii) as a daily dose during exposure of the subject to the HIV; and
iii) as a single or double dose within about 48 hours after final exposure of the subject to the HIV.

In some embodiments, the compound of Formula (I) or Formula (II), or a pharmaceutically acceptable salt thereof, is administered:
i) as a single or double dose about 2 hours before exposure of the subject to the HIV;
ii) as a daily dose during exposure of the subject to the HIV; and
iii) as a single or double dose within about 48 hours after final exposure of the subject to the HIV.

In some embodiments, the compound of Formula (I) or Formula (II), or a pharmaceutically acceptable salt thereof, is administered:
i) as a single or double dose within about 2 hours before exposure of the subject to the HIV;
ii) as a daily dose during exposure of the subject to the HIV;
iii) as a single or double dose within about 48 hours after exposure of the subject to the HIV; and
iv) as a single or double dose between about 48 hours and about 72 hours after final exposure of the subject to the HIV.

In some embodiments, the compound of Formula (I) or Formula (II), or a pharmaceutically acceptable salt thereof, is administered:
i) as a first administration (*e*.*g*., a single or double dose) within or at about 24 hours after exposure of the subject to the HIV; and
ii) as a second administration (*e*.*g*., a single or double dose) between about 24 hours and about 48 hours after the first administration.

In some embodiments, the compound of Formula (I) or Formula (II), or a pharmaceutically acceptable salt thereof, is administered:
i) as a first administration (*e*.*g*., a single or double dose) within or at about 24 hours after exposure of the subject to the HIV; and
ii) as a second administration (*e*.*g*., a single or double dose) within or at about 24 hours after the first administration.

In some embodiments, *e*.*g*., when administered as part of a PrEP regimen or as part of a PEP regimen, the compound of Formula (I) or Formula (II), or a pharmaceutically acceptable salt thereof, is administered 1 hour to 10 days, 1 hour to 7 days, 1 hour to 5 days, 1 to 72 hours, 1 to 48 hours, 1 to 36 hours, 1 to 24 hours, or 1 to 12 hours following an event that would increase the individual's risk of acquiring HIV (*e*.*g*., following sex or other exposure to the HIV virus). In certain embodiments, e.g., when administered as PEP, the compound of Formula (I) or Formula (II), or a pharmaceutically acceptable salt thereof, is administered for 7 days, 14 days, 21 days, 28 days, 30 days, or 45 days following an event that would increase the individual risk of acquiring HIV (*e*.*g*., following sex or other exposure to the HIV virus). In certain embodiments, *e*.*g*., when administered as PEP, the compound of Formula (I) or Formula (II), or a pharmaceutically acceptable salt thereof, is administered for 30 days following an event that would increase the individual risk of acquiring HIV (*e*.*g*., following sex or other exposure to the HIV virus). In certain embodiments, the compound of Formula (I) or Formula (II), or a pharmaceutically acceptable salt thereof, is administered less than 1 hour, 2 hours, 3 hours, 4, hours, 5 hours, 6 hours, 7 hours, 8 hours, 9 hours, 12 hours, 18 hours, 24 hours, 36 hours, or 48 hours following an event that would increase the individual's risk of acquiring HIV (*e*.*g*., following sex or other exposure to the HIV virus). In certain embodiments, the compound of Formula (I) or Formula (II), or a pharmaceutically acceptable salt thereof, is administered for 1 day, 2 days, 3, days 4 days, or 5 days following an event that would increase the individual's risk of acquiring HIV (*e*.*g*., following sex or other exposure to the HIV virus). In certain embodiments, when the compound of Formula (I) or Formula (II), or a pharmaceutically acceptable salt thereof, is administered following to an event that would increase the individual's risk of acquiring HIV, it is administered daily following the event. In certain embodiments, when the compound of Formula (I) or Formula (II), or a pharmaceutically acceptable salt thereof, is administered following an event that would increase the individual's risk of acquiring HIV, it is administered one to three times following the event. In certain embodiments, when the compound of Formula (I) or Formula (II), or a pharmaceutically acceptable salt thereof, is administered following an event that would increase the individual's risk of acquiring HIV, it is administered once following the event.

In certain embodiments, when the compound of Formula (I) or Formula (II), or a pharmaceutically acceptable salt thereof, is administered following an event that would increase the individual's risk of acquiring HIV, it is administered twice following the event (*e*.*g*., a first administration within a 24 hour period following the event and a second administration about 24 hours following the first administration).

In some embodiments, the compound of Formula (I) or Formula (II), or a pharmaceutically acceptable salt thereof, is administered during exposure of the subject to the HIV (*e*.*g*., during a period of sexual activity with sex partner known to be HIV positive). In some embodiments, the compound of Formula (I) or Formula (II), or a pharmaceutically acceptable salt thereof, is administered after exposure (*e*.*g*., after final exposure) of the subject to the HIV (*e*.*g*., after a period of sexual activity with sex partner known to be HIV positive). In some embodiments, the compound of Formula (I) or Formula (II), or a pharmaceutically acceptable salt thereof, is administered from about 1 hour to about 72 hours after exposure (*e*.*g*., after final exposure) of the subject to the HIV. In some embodiments, the compound of Formula (I) or Formula (II), or a pharmaceutically acceptable salt thereof, is administered from about 1 hour to about 24 hours after exposure (*e*.*g*., after final exposure) of the subject to the HIV. In some embodiments, the compound of Formula (I) or Formula (II), or a pharmaceutically acceptable salt thereof, is administered from about 24 hours to about 72 hours after exposure (*e*.*g*., after final exposure) of the subject to the HIV.

In some embodiments, *e*.*g*., when administered as PrEP, the compound of Formula (I) or Formula (II), or a pharmaceutically acceptable salt thereof, is administered prior to an event that would increase the individual's risk of acquiring HIV (*e*.*g*., prior to sexual activity), and following the event. For example, in certain embodiments, *e*.*g*., when administered as PrEP, the compound of Formula (I) or Formula (II), or a pharmaceutically acceptable salt thereof, is administered 1 to 72 hours, 1 to 48 hours, 1 to 24 hours, or 1 to 12 hours prior to an event that would increase the individual's risk of acquiring HIV (*e*.*g*., prior to sexual activity) and 1 to 48 hours, 1 to 36 hours, 1 to 24 hours, or 1 to 12 hours following the event. For example, in some embodiments, one or more (*e*.*g*., one, two, or three) dosages of the compound of Formula (I) or Formula (II), or a pharmaceutically acceptable salt thereof, are administered one to three days prior to an event that would increase the individual's risk of acquiring HIV (*e*.*g*., prior to sex) and once per day for a period of one to five days following the event. In some embodiments, one or more (*e*.*g*., one, two, or three) dosages of the compound of Formula (I) or Formula (II), or a pharmaceutically acceptable salt thereof, are administered 1 to 24 hours prior to an event that would increase the individual's risk of acquiring HIV (*e*.*g*., prior to sexual activity) and one or more times (*e*.*g*., one, two, or three times) 1 to 48 hours following the event. In some embodiments, the compound of Formula (I) or Formula (II), or a pharmaceutically acceptable salt thereof, is administered once per week, twice per week, three times per week, four times per week, or five times per week and one or more times (*e*.*g*., one, two, or three times) 1 to 48 hours following an event that would increase the individual's risk of acquiring HIV (*e*.*g*., prior to sex). In some embodiments, the compound of Formula (I) or Formula (II), or a pharmaceutically acceptable salt thereof, is administered twice per week, and once following an event that increases the individual's risk of acquiring HIV (*e*.*g*., one tablet within 24 hours of exposure, such as following sex).

In some embodiments, the present invention further provides a method of preventing an HIV infection in a subject, comprising administering to the subject the compound of Formula (I) or Formula (II), or a pharmaceutically acceptable salt thereof (*e*.*g*., a sodium salt), in a dosage of about 10 mg/day to about 200 mg/day, wherein the administration is performed about 72 hours to about 1 hour before exposure of the subject to the HIV or about 1 hour to about 72 hours after exposure of the subject to the HIV.

In some embodiments, the present application further provides a method of reducing the risk of acquiring HIV in a subject, comprising administering to the subject the compound of Formula (I) or Formula (II), or a pharmaceutically acceptable salt thereof, (*e*.*g*., a sodium salt) in a dosage of about 10 mg/day to about 200 mg/day, wherein the administration is performed about 72 hours to about 1 hour before exposure of the subject to the HIV or about 1 hour to about 72 hours after exposure of the subject to the HIV.

In some embodiments, the present invention further provides a method of preventing an HIV infection in a subject, comprising administering to the subject the compound of Formula (I) or Formula (II), or a pharmaceutically acceptable salt thereof (*e*.*g*., a sodium salt), in a dosage of about 10 mg/day to about 200 mg/day, wherein a first administration is performed within about 24 hours after exposure of the subject to the HIV and a second administration is performed within or at about 24 hours after the first administration. Accordingly, in some embodiments, the first administration is performed about 6 hours after exposure of the subject to HIV and the second administration is performed about 30 hours after exposure of the subject to HIV. In some embodiments, the first administration is performed about 12 hours after exposure of the subject to HIV and the second administration is performed about 36 hours after exposure of the subject to HIV. In some embodiments, the first administration is performed about 24 hours after exposure of the subject to HIV and the second administration is performed about 48 hours after exposure of the subject to HIV.

In some embodiments, the present application further provides a method of reducing the risk of acquiring HIV in a subject, comprising administering to the subject the compound of Formula (I) or Formula (II), or a pharmaceutically acceptable salt thereof, (*e*.*g*., a sodium salt) in a dosage of about 10 mg/day to about 200 mg/day, wherein a first administration is performed within about 24 hours after exposure of the subject to the HIV and a second administration is performed within or at about 24 hours after the first administration. Accordingly, in some embodiments, the first administration is performed about 6 hours after exposure of the subject to HIV and the second administration is performed about 30 hours after exposure of the subject to HIV. In some embodiments, the first administration is performed about 12 hours after exposure of the subject to HIV and the second administration is performed about 36 hours after exposure of the subject to HIV. In some embodiments, the first administration is performed about 24 hours after exposure of the subject to HIV and the second administration is performed about 48 hours after exposure of the subject to HIV.

In some embodiments, the present invention further provides a method of preventing an HIV infection in a subject, comprising administering to the subject the compound of Formula (I) or Formula (II), or a pharmaceutically acceptable salt thereof (*e*.*g*., a sodium salt), in a dosage of about 10 mg/day to about 100 mg/day, wherein the administration is performed about 72 hours to about 1 hour before exposure of the subject to the HIV or about 1 hour to about 72 hours after exposure of the subject to the HIV.

In some embodiments, the present application further provides a method of reducing the risk of acquiring HIV in a subject, comprising administering to the subject the compound of Formula (I) or Formula (II), or a pharmaceutically acceptable salt thereof, (*e*.*g*., a sodium salt) in a dosage of about 10 mg/day to about 100 mg/day, wherein the administration is performed about 72 hours to about 1 hour before exposure of the subject to the HIV or about 1 hour to about 72 hours after exposure of the subject to the HIV.

In some embodiments, the present invention further provides a method of preventing an HIV infection in a subject, comprising administering to the subject the compound of Formula (I) or Formula (II), or a pharmaceutically acceptable salt thereof (*e*.*g*., a sodium salt), in a dosage of about 100 mg/day, wherein the administration is performed about 72 hours to about 1 hour before exposure of the subject to the HIV or about 1 hour to about 72 hours after exposure of the subject to the HIV.

In some embodiments, the present application further provides a method of reducing the risk of acquiring HIV in a subject, comprising administering to the subject the compound of Formula (I) or Formula (II), or a pharmaceutically acceptable salt thereof, (*e*.*g*., a sodium salt) in a dosage of about 100 mg/day, wherein the administration is performed about 72 hours to about 1 hour before exposure of the subject to the HIV or about 1 hour to about 72 hours after exposure of the subject to the HIV.

In some embodiments, the present invention further provides a method of preventing an HIV infection in a subject, comprising administering to the subject the compound of Formula (I) or Formula (II), or a pharmaceutically acceptable salt thereof (*e*.*g*., a sodium salt), in a dosage of about 100 mg/day, wherein a first administration is performed within about 24 hours after exposure of the subject to the HIV and a second administration is performed within or at about 24 hours after the first administration. Accordingly, in some embodiments, the first administration is performed about 6 hours after exposure of the subject to HIV and the second administration is performed about 30 hours after exposure of the subject to HIV. In some embodiments, the first administration is performed about 12 hours after exposure of the subject to HIV and the second administration is performed about 36 hours after exposure of the subject to HIV. In some embodiments, the first administration is performed about 24 hours after exposure of the subject to HIV and the second administration is performed about 48 hours after exposure of the subject to HIV.

In some embodiments, the present application further provides a method of reducing the risk of acquiring HIV in a subject, comprising administering to the subject the compound of Formula (I) or Formula (II), or a pharmaceutically acceptable salt thereof, (*e*.*g*., a sodium salt) in a dosage of about 100 mg/day, wherein a first administration is performed within about 24 hours after exposure of the subject to the HIV and a second administration is performed within or at about 24 hours after the first administration. Accordingly, in some embodiments, the first administration is performed about 6 hours after exposure of the subject to HIV and the second administration is performed about 30 hours after exposure of the subject to HIV. In some embodiments, the first administration is performed about 12 hours after exposure of the subject to HIV and the second administration is performed about 36 hours after exposure of the subject to HIV. In some embodiments, the first administration is performed about 24 hours after exposure of the subject to HIV and the second administration is performed about 48 hours after exposure of the subject to HIV.

In some embodiments, methods for reducing the risk of acquiring HIV (*e*.*g*., HIV-1 and/or HIV-2) comprise administration of the compound of Formula (I) or Formula (II), or a pharmaceutically acceptable salt thereof, in combination with safer sex practices. In certain embodiments, methods for reducing the risk of acquiring HIV (*e*.*g*., HIV-1 and/or HIV-2) comprise administration to an individual at risk of acquiring HIV. Examples of individuals at high risk for acquiring HIV include, without limitation, an individual who is at risk of sexual transmission of HIV.

In some embodiments, the reduction in risk of acquiring HIV is at least about 40%, 50%, 60%, 70%, 80%, 90%, or 95%. In some embodiments, the reduction in risk of acquiring HIV is at least about 75%. In some embodiments, the reduction in risk of acquiring HIV is about 80%, 85%, or 90%.

The present invention further includes salts of the compound of Formula (I) or Formula (II), such as pharmaceutically acceptable salts. A salt generally refers to a derivative of a disclosed compound wherein the parent compound is modified by converting an existing acid or base moiety to its salt form. A pharmaceutically acceptable salt is one that, within the scope of sound medical judgment, is suitable for use in contact with the tissues of human beings and animals without excessive toxicity, irritation, allergic response, or other problem or complication, commensurate with a reasonable benefit/risk ratio. Examples of pharmaceutically acceptable salts include, but are not limited to, mineral or organic acid salts of basic residues such as amines; alkali or organic salts of acidic residues such as carboxylic acids; and the like. The pharmaceutically acceptable salts of the present invention include the conventional non-toxic salts of the parent compound formed, for example, from non-toxic inorganic or organic acids. The pharmaceutically acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid. Lists of suitable salts are found in Remington's Pharmaceutical Sciences, 17th ed., Mack Publishing Company, Easton, Pa., 1985, p. 1418 and Journal of Pharmaceutical Science, 66, 2 (1977), each of which is incorporated herein by reference in its entirety. In some embodiments, the salt is a sodium salt.

The compound of Formula (I) or Formula (II), or its salt, can be present in a composition where the composition includes at least one compound other than the compound of Formula (I) or Formula (II) or salt of the invention. In some embodiments, the composition comprises bictegravir, or a salt thereof, and one or more additional compounds (*e*.*g*., one or more additional therapeutic compounds), or salts thereof. In some embodiments, the composition comprises bictegravir, or a salt thereof; cobicistat, or a salt thereof; and one or more additional compounds (*e*.*g*., one or more additional therapeutic compounds), or salts thereof. In some embodiments, the composition comprises elvitegravir, or a salt thereof, and one or more additional compounds (*e*.*g*., one or more additional therapeutic compounds), or salts thereof. In some embodiments, the composition comprises elvitegravir, or a salt thereof; cobicistat, or a salt thereof; and one or more additional compounds (*e*.*g*., one or more additional therapeutic compounds), or salts thereof. Compositions can include mixtures containing the compound of Formula (I) or Formula (II), or salt thereof, and one or more solvents, substrates, carriers, etc. In some embodiments, the composition comprises the compound of Formula (I) or Formula (II), or salt thereof, in an amount greater than about 25% by weight, for example, greater than about 25% by weight, greater than about 50% by weight, greater than about 75% by weight, greater than about 80% by weight, greater than about 90% by weight, or greater than about 95% by weight.

The present invention further includes pharmaceutical compositions comprising the compound of Formula (I) or Formula (II), or pharmaceutically acceptable salt thereof, and at least one pharmaceutically acceptable carrier. As used herein, "pharmaceutically acceptable carrier" is meant to refer to any adjuvant, carrier, excipient, glidant, sweetening agent, diluent, preservative, dye/colorant, flavor enhancer, surfactant, wetting agent, dispersing agent, suspending agent, stabilizer, isotonic agent, solvent, or emulsifier which has been approved by the United States Food and Drug Administration as being acceptable for use in humans or domestic animals.

Administration of the compound of Formula (I) or Formula (II), or a pharmaceutically acceptable salt thereof, can be carried out via any of the accepted modes of administration of agents for serving similar utilities. The pharmaceutical compositions of the invention can be prepared by combining the compound of Formula (I) or Formula (II), or a pharmaceutically acceptable salt thereof, with an appropriate pharmaceutically acceptable carrier and, in specific embodiments, are formulated into preparations in solid, semi solid, liquid or gaseous forms, such as tablets, capsules, powders, granules, ointments, solutions, suppositories, injections, inhalants, gels, microspheres, and aerosols. Exemplary routes of administering such pharmaceutical compositions include, without limitation, oral, topical, transdermal, inhalation, parenteral, sublingual, buccal, rectal, vaginal, and intranasal. In a some embodiments, pharmaceutical compositions of the invention are tablets. In some embodiments, pharmaceutical compositions of the invention are injection (*e*.*g*., intramuscular (IM) or intraperitoneal (IP)). Pharmaceutical compositions of the invention are formulated so as to allow the active ingredients contained therein to be bioavailable upon administration of the composition to a subject. Compositions that will be administered to a subject take the form of one or more dosage units, where for example, a tablet may be a single dosage unit, and a container of the compound of Formula (I) or Formula (II), or a pharmaceutically acceptable salt thereof, in aerosol form may hold a plurality of dosage units. Actual methods of preparing such dosage forms are known, or will be apparent, to those skilled in this art; for example, see Remington: The Science and Practice of Pharmacy, 20th Edition (Philadelphia College of Pharmacy and Science, 2000). The composition to be administered will, in any event, contain a therapeutically effective amount of the compound of Formula (I) or Formula (II), or a pharmaceutically acceptable salt thereof, for prevention of an HIV infection or reducing the risk of acquiring HIV, as described herein.

In some embodiments, the compound of Formula (I) or Formula (II), or a pharmaceutically acceptable salt thereof, is administered orally. In some embodiments, the compound of Formula (I) or Formula (II), or a pharmaceutically acceptable salt thereof, is administered parenterally. Parenteral administration includes, but is not limited to, intravenous, intraarterial, subcutaneous, intraperitoneal, intramuscular, intracranial, transdermal, and vaginal administration. Parenteral administration can be administered, for example, in the form of a single bolus dose or by a continuous perfusion pump. In some embodiments, the compound of Formula (I) or Formula (II), or a pharmaceutically acceptable salt thereof, is administered to the subject through a medical device. Exemplary medical devices include, but are not limited to, a patch (*e*.*g*., a transdermal patch), an implantable device (*e*.*g*., an implantable device for metered or sustained release of an active agent; a subdermal device), a syringe, a contraceptive device (*e*.*g*., a vaginal ring, an intrauterine device), and the like..

The pharmaceutical compositions disclosed herein can be prepared by methodologies well known in the pharmaceutical art. For example, in certain embodiments, a pharmaceutical composition intended to be administered by injection can prepared by combining the compound of Formula (I) or Formula (II), or a pharmaceutically acceptable salt thereof, with sterile, distilled water so as to form a solution. In some embodiments, a surfactant is added to facilitate the formation of a homogeneous solution or suspension. Surfactants are compounds that non-covalently interact with the compound of Formula (I) or Formula (II), or a pharmaceutically acceptable salt thereof, so as to facilitate dissolution or homogeneous suspension of the compound in the aqueous delivery system.

The terms "effective amount" or "therapeutically effective amount" refer to an amount of the compound of Formula (I) or Formula (II), or other anti-HIV agent, or a pharmaceutically acceptable salt thereof, which when administered to a subject in need thereof, is sufficient to effect preventing an HIV infection or reducing the risk of contracting HIV infection, as described herein. Such an amount would be sufficient to elicit the biological or medical response of a tissue system, or subject that is sought by a researcher or clinician. The amount of the compound of Formula (I) or Formula (II) or other anti-HIV agent which constitutes a therapeutically effective amount will vary depending on such factors as the compound, salt, or composition used for administration, the time of administration, the route of administration, the rate of excretion of the compound, the duration of the treatment, the type of disease-state or disorder being treated and its severity, drugs used in combination with or coincidentally with the compound of Formula (I) or Formula (II), and the age, body weight, general health, sex and diet of the subject. Such a therapeutically effective amount can be determined routinely by one of ordinary skill in the art having regard to their own knowledge, the state of the art, and this disclosure.

The term "subject" is meant to refer to a human or other mammals such as laboratory animals and household pets (*e*.*g*., cats, dogs, swine, cattle, sheep, goats, horses, rabbits), and non-domestic animals such as non-human primates, mammalian wildlife, and the like, that are in need of therapeutic or preventative treatment for a viral infection, such as HIV infection.

### Combination Therapies

One or more additional therapeutic agents can be used in combination with the compounds, salts, and compositions of the present invention for preventing an HIV infection in a subject (*e*.*g*., in a human subject). In some embodiments, the composition of the invention comprises the compound of Formula (I) or Formula (II), or a pharmaceutically acceptable salt thereof, and one or more additional therapeutic agents. In some embodiments, the composition of the invention comprises bictegravir, or a pharmaceutically acceptable salt thereof, and one to three additional therapeutic agents (*e*.*g*., one to three anti-HIV agents). In some embodiments, the composition of the invention comprises bictegravir, or a pharmaceutically acceptable salt thereof, cobicistat, or a pharmaceutically acceptable salt thereof, and one to three additional therapeutic agents (*e.g.*, one to three anti-HIV agents). In some embodiments, the composition of the invention comprises elvitegravir, or a pharmaceutically acceptable salt thereof, and one to three additional therapeutic agents (*e*.*g*., one to three anti-HIV agents). In some embodiments, the composition of the invention comprises elvitegravir, or a pharmaceutically acceptable salt thereof, cobicistat, or a pharmaceutically acceptable salt thereof, and one to three additional therapeutic agents (*e*.*g*., one to three anti-HIV agents).

In the above embodiments, the additional therapeutic agent may be an anti-HIV agent. For example, in some embodiments, the additional therapeutic agent is selected from the group consisting of HIV protease inhibitors, HIV non-nucleoside inhibitors of reverse transcriptase, HIV nucleoside inhibitors of reverse transcriptase, HIV nucleotide inhibitors of reverse transcriptase, HIV integrase inhibitors, HIV non-catalytic site (or allosteric) integrase inhibitors, entry inhibitors (*e*.*g*., CCR5 inhibitors, gp41 inhibitors (i.e., fusion inhibitors) and CD4 attachment inhibitors), CXCR4 inhibitors, gp120 inhibitors, G6PD and NADH-oxidase inhibitors, compounds that target the HIV capsid ("capsid inhibitors"; *e*.*g*., capsid polymerization inhibitors or capsid disrupting compounds such as those disclosed in WO 2013/006738 (Gilead Sciences), US 2013/0165489 (University of Pennsylvania), and WO 2013/006792 (Pharma Resources), pharmacokinetic enhancers, and other drugs for treating HIV, and combinations thereof. In some embodiments, the anti-HIV agent is an HIV protease inhibitor, an HIV non-nucleoside inhibitor of reverse transcriptase, an HIV nucleoside inhibitor of reverse transcriptase, an HIV nucleotide inhibitors of reverse transcriptase, a pharmacokinetic enhancer, or combination thereof. In some embodiments, the anti-HIV agent is an HIV nucleoside inhibitor of reverse transcriptase, an HIV nucleotide inhibitors of reverse, or combination thereof. In some embodiments, each of the one or more additional therapeutic agents is an anti-HIV agent.

In further embodiments, the additional therapeutic agent is selected from one or more of:
(1) HIV protease inhibitors selected from the group consisting of amprenavir, atazanavir, fosamprenavir, indinavir, lopinavir, ritonavir, nelfinavir, saquinavir, tipranavir, brecanavir, darunavir, TMC-126, TMC-114, mozenavir (DMP-450), JE-2147 (AG1776), L-756423, RO0334649, KNI-272, DPC-681, DPC-684, GW640385X, DG17, PPL-100, DG35, and AG 1859;
(2) HIV non-nucleoside or non-nucleotide inhibitors of reverse transcriptase selected from the group consisting of capravirine, emivirine, delaviridine, efavirenz, nevirapine, (+) calanolide A, etravirine, GW5634, DPC-083, DPC-961, DPC-963, MIV-150, TMC-120, rilpivirene, BILR 355 BS, VRX 840773, lersivirine (UK-453061), RDEA806, KM023 and MK-1439;
(3) HIV nucleoside inhibitors of reverse transcriptase selected from the group consisting of zidovudine, emtricitabine, didanosine, stavudine, zalcitabine, lamivudine, abacavir, amdoxovir, elvucitabine, alovudine, MIV-210, ±-FTC, D-d4FC, phosphazide, fozivudine tidoxil, apricitibine (AVX754), KP-1461, GS-9131 (Gilead Sciences) and fosalvudine tidoxil (formerly HDP 99.0003);
(4) HIV nucleotide inhibitors of reverse transcriptase selected from the group consisting of tenofovir, tenofovir disoproxil fumarate, tenofovir alafenamide (Gilead Sciences), GS-7340 (Gilead Sciences), GS-9148 (Gilead Sciences), adefovir, adefovir dipivoxil, CMX-001 (Chimerix) or CMX-157 (Chimerix);
(5) HIV integrase inhibitors selected from the group consisting of raltegravir, elvitegravir, dolutegravir, cabotegravir, curcumin, derivatives of curcumin, chicoric acid, derivatives of chicoric acid, 3,5-dicaffeoylquinic acid, derivatives of 3,5-dicaffeoylquinic acid, aurintricarboxylic acid, derivatives of aurintricarboxylic acid, caffeic acid phenethyl ester, derivatives of caffeic acid phenethyl ester, tyrphostin, derivatives of tyrphostin, quercetin, derivatives of quercetin, S-1360, AR-177, L-870812, and L-870810, BMS-538158, GSK364735C, BMS-707035, MK-2048, BA 011, and GSK-744;
(6) HIV non-catalytic site, or allosteric, integrase inhibitors (NCINI) including, but not limited to, BI-224436, CX0516, CX05045, CX14442, compounds disclosed in WO 2009/062285 (Boehringer Ingelheim), WO 2010/130034 (Boehringer Ingelheim), WO 2013/159064 (Gilead Sciences), WO 2012/145728 (Gilead Sciences), WO 2012/003497 (Gilead Sciences), WO 2012/003498 (Gilead Sciences) each of which is incorporated by references in its entirety herein;
(7) gp41 inhibitors selected from the group consisting of enfuvirtide, sifuvirtide, albuvirtide, FB006M, and TRI-1144;
(8) the CXCR4 inhibitor AMD-070;
(9) the entry inhibitor SP01A;
(10) the gp120 inhibitor BMS-488043;
(11) the G6PD and NADH-oxidase inhibitor immunitin;
(12) CCR5 inhibitors selected from the group consisting of aplaviroc, vicriviroc, maraviroc, cenicriviroc, PRO-140, INCB15050, PF-232798 (Pfizer), and CCR5mAb004;
(13) CD4 attachment inhibitors selected from the group consisting of ibalizumab (TMB-355) and BMS-068 (BMS-663068);
(14) pharmacokinetic enhancers selected from the group consisting of ritonavir, cobicistat and SPI-452; and
(15) other drugs for treating HIV selected from the group consisting of BAS-100, SPI-452, REP 9, SP-01A, TNX-355, DES6, ODN-93, ODN-112, VGV-1, PA-457 (bevirimat), HRG214, VGX-410, KD-247, AMZ 0026, CYT 99007A-221 HIV, DEBIO-025, BAY 50-4798, MDX010 (ipilimumab), PBS 119, ALG 889, and PA-1050040 (PA-040),
and combinations thereof.

In some embodiments, the compound of Formula (I) or Formula (II), or a pharmaceutically acceptable salt thereof, is combined with one, two, three, or more additional therapeutic agents. The one, two, three, or more additional therapeutic agents can be different therapeutic agents selected from the same class of therapeutic agents, or they can be selected from different classes of therapeutic agents. In a specific embodiment, the compound of Formula (I) or Formula (II), or a pharmaceutically acceptable salt thereof, is combined with an HIV nucleotide or nucleoside inhibitor of reverse transcriptase and an HIV non-nucleoside inhibitor of reverse transcriptase. In another specific embodiment, the compound of Formula (I) or Formula (II), or a pharmaceutically acceptable salt thereof, is combined with an HIV nucleotide or nucleoside inhibitor of reverse transcriptase, and an HIV protease inhibiting compound. In a further embodiment, the compound of Formula (I) or Formula (II), or a pharmaceutically acceptable salt thereof, is combined with an HIV nucleotide or nucleoside inhibitor of reverse transcriptase, an HIV non-nucleoside inhibitor of reverse transcriptase, and an HIV protease inhibiting compound. In an additional embodiment, the compound of Formula (I) or Formula (II), or a pharmaceutically acceptable salt thereof, is combined with an HIV nucleotide or nucleoside inhibitor of reverse transcriptase, an HIV non-nucleoside inhibitor of reverse transcriptase, and a pharmacokinetic enhancer.

In some embodiments, the compound of Formula (I) or Formula (II), or a pharmaceutically acceptable salt thereof, is administered in combination with an additional therapeutic agent which is emtricitabine, or a pharmaceutically acceptable salt thereof. In some embodiments, the additional therapeutic agent is emtricitabine.

In some embodiments, the emtricitabine, or a pharmaceutically acceptable salt thereof, is administered in a dosage of about 10 mg/day to about 500 mg/day, for example, about 10 mg/day, about 50 mg/day, about 100 mg/day, about 150 mg/day, about 200 mg/day, about 250 mg/day, about 300 mg/day, about 350 mg/day, about 400 mg/day, about 450 mg/day, or about 500 mg/day. In some embodiments, the emtricitabine, or a pharmaceutically acceptable salt thereof, is administered in a dosage of about 100 mg/day to about 300 mg/day. In some embodiments, the emtricitabine, or a pharmaceutically acceptable salt thereof, is administered in a dosage of about 175 mg/day to about 225 mg/day. In some embodiments, the emtricitabine, or a pharmaceutically acceptable salt thereof, is administered in a dosage of about 190 mg/day to about 210 mg/day In some embodiments, the emtricitabine, or a pharmaceutically acceptable salt thereof, is administered in a dosage of about 200 mg/day.

In some embodiments, the compound of Formula (I) or Formula (II), or a pharmaceutically acceptable salt thereof, is administered in combination with an additional therapeutic agent selected from tenofovir alafenamide, or a pharmaceutically acceptable salt thereof, and tenofovir disoproxil, or a pharmaceutically acceptable salt thereof.

In some embodiments, the additional therapeutic agent is tenofovir alafenamide, or a pharmaceutically acceptable salt thereof. In some embodiments, the tenofovir alafenamide, or a pharmaceutically acceptable salt thereof, is administered in a dosage of about 1 mg/day to about 100 mg/day, for example, about 1 mg/day, about 10 mg/day, about 25 mg/day, about 50 mg/day, about 75 mg/day, or about 100 mg/day. In some embodiments, the tenofovir alafenamide, or a pharmaceutically acceptable salt thereof, is administered in a dosage of about 10 mg/day to about 50 mg/day. In some embodiments, the tenofovir alafenamide, or a pharmaceutically acceptable salt thereof, is administered in a dosage of about 20 mg/day to about 30 mg/day. In some embodiments, the tenofovir alafenamide, or a pharmaceutically acceptable salt thereof, is administered in a dosage of about 25 mg/day. In some embodiments, the tenofovir alafenamide is tenofovir alafenamide hemifumarate. In some embodiments, the tenofovir alafenamide hemifumarate is administered in a dosage of about 28 mg/day.

In some embodiments, the additional therapeutic agent is tenofovir disoproxil, or a pharmaceutically acceptable salt thereof. In some embodiments, the tenofovir disoproxil, or a pharmaceutically acceptable salt thereof, is administered in a dosage of about 1 mg/day to about 500 mg/day, for example, about 1 mg/day, about 10 mg/day, about 25 mg/day, about 50 mg/day, about 75 mg/day, about 100 mg/day, about 150 mg/day, about 200 mg/day, about 250 mg/day, about 300 mg/day, about 350 mg/day, about 400 mg/day, about 450 mg/day, or about 500 mg/day. In some embodiments, the tenofovir disoproxil, or a pharmaceutically acceptable salt thereof, is administered in a dosage of about 123 mg/day, about 163 mg/day, about 204 mg/day, or about 245 mg/day. In some embodiments, the tenofovir disoproxil, or a pharmaceutically acceptable salt thereof, is administered in a dosage of about 10 mg/day to about 500 mg/day. In some embodiments, the tenofovir disoproxil, or a pharmaceutically acceptable salt thereof, is administered in a dosage of about 20 mg/day to about 300 mg/day. In some embodiments, the tenofovir disoproxil, or a pharmaceutically acceptable salt thereof, is administered in a dosage of about 10 mg/day to about 50 mg/day. In some embodiments, the tenofovir disoproxil, or a pharmaceutically acceptable salt thereof, is administered in a dosage of about 20 mg/day to about 30 mg/day. In some embodiments, the tenofovir disoproxil, or a pharmaceutically acceptable salt thereof, is administered in a dosage of about 25 mg/day. In some embodiments, the tenofovir disoproxil is tenofovir disoproxil fumarate. In some embodiments, the tenofovir disoproxil fumarate is administered in a dosage of about 150 mg/day, about 200 mg/day, about 250 mg/day, or about 300 mg/day.

In some embodiments, bictegravir, or a pharmaceutically acceptable salt thereof, is administered in combination with a first additional therapeutic agent which is emtricitabine, or a pharmaceutically acceptable salt thereof, and a second additional therapeutic agent which is tenofovir alafenamide, or a pharmaceutically acceptable salt thereof. In some embodiments, the first additional therapeutic agent is emtricitabine and the second additional therapeutic agent is tenofovir alafenamide hemifumarate.

In some embodiments, elvitegravir, or a pharmaceutically acceptable salt thereof, is administered in combination with a first additional therapeutic agent which is emtricitabine, or a pharmaceutically acceptable salt thereof, a second additional therapeutic agent which is tenofovir alafenamide, or a pharmaceutically acceptable salt thereof. In some embodiments, the first additional therapeutic agent is emtricitabine and the second additional therapeutic agent is tenofovir alafenamide hemifumarate.

In some embodiments, bictegravir, or a pharmaceutically acceptable salt thereof, is administered in combination with a first additional therapeutic agent which is emtricitabine, or a pharmaceutically acceptable salt thereof, a second additional therapeutic agent which is tenofovir alafenamide, or a pharmaceutically acceptable salt thereof, and a third additional therapeutic agent which is cobicistat, or a pharmaceutically acceptable salt thereof. In some embodiments, the first additional therapeutic agent is emtricitabine, the second additional therapeutic agent is tenofovir alafenamide hemifumarate, and the third additional therapeutic agent is cobicistat.

In some embodiments, elvitegravir, or a pharmaceutically acceptable salt thereof, is administered in combination with a first additional therapeutic agent which is emtricitabine, or a pharmaceutically acceptable salt thereof, a second additional therapeutic agent which is tenofovir alafenamide, or a pharmaceutically acceptable salt thereof, and a third additional therapeutic agent which is cobicistat, or a pharmaceutically acceptable salt thereof. In some embodiments, the first additional therapeutic agent is emtricitabine, the second additional therapeutic agent is tenofovir alafenamide hemifumarate, and the third additional therapeutic agent is cobicistat.

In some embodiments, bictegravir, or a pharmaceutically acceptable salt thereof, is administered in combination with a first additional therapeutic agent which is emtricitabine, or a pharmaceutically acceptable salt thereof, and a second additional therapeutic agent which is tenofovir disoproxil, or a pharmaceutically acceptable salt thereof. In some embodiments, the first additional therapeutic agent is emtricitabine and the second additional therapeutic agent is tenofovir disoproxil fumarate.

In some embodiments, elvitegravir, or a pharmaceutically acceptable salt thereof, is administered in combination with a first additional therapeutic agent which is emtricitabine, or a pharmaceutically acceptable salt thereof, a second additional therapeutic agent which is tenofovir disoproxil, or a pharmaceutically acceptable salt thereof. In some embodiments, the first additional therapeutic agent is emtricitabine and the second additional therapeutic agent is tenofovir disoproxil fumarate.

In some embodiments, bictegravir, or a pharmaceutically acceptable salt thereof, is administered in combination with a first additional therapeutic agent which is emtricitabine, or a pharmaceutically acceptable salt thereof, a second additional therapeutic agent which is tenofovir disoproxil, or a pharmaceutically acceptable salt thereof, and a third additional therapeutic agent which is cobicistat, or a pharmaceutically acceptable salt thereof. In some embodiments, the first additional therapeutic agent is emtricitabine, the second additional therapeutic agent is tenofovir disoproxil fumarate, and the third additional therapeutic agent is cobicistat.

In some embodiments, elvitegravir, or a pharmaceutically acceptable salt thereof, is administered in combination with a first additional therapeutic agent which is emtricitabine, or a pharmaceutically acceptable salt thereof, a second additional therapeutic agent which is tenofovir disoproxil, or a pharmaceutically acceptable salt thereof, and a third additional therapeutic agent which is cobicistat, or a pharmaceutically acceptable salt thereof. In some embodiments, the first additional therapeutic agent is emtricitabine, the second additional therapeutic agent is tenofovir disoproxil fumarate, and the third additional therapeutic agent is cobicistat.

The present invention further provides a method of preventing an HIV infection in a subject, comprising administering to the subject:
(a) bictegravir, or a pharmaceutically acceptable salt thereof, in a dosage of about 10 mg/day to about 200 mg/day;
(b) emtricitabine, or a pharmaceutically acceptable salt thereof, in a dosage of about 100 mg/day to about 400 mg/day; and
(c) tenofovir alafenamide, or a pharmaceutically acceptable salt thereof, in a dosage of about 10 mg/day to about 50 mg/day. In some embodiments, the administration is performed one to three times between about 72 hours to about 1 hour before exposure of the subject to the HIV and/or one to three times about 1 hour to about 72 hours after exposure of the subject to the HIV. In some embodiments, the administration is performed one to three times about 1 hour to about 72 hours after exposure of the subject to the HIV.

The present invention further provides a method of preventing an HIV infection in a subject, comprising administering to the subject:
(a) bictegravir sodium, in a dosage of about 10 mg/day to about 200 mg/day;
(b) emtricitabine in a dosage of about 100 mg/day to about 400 mg/day; and
(c) tenofovir alafenamide hemifumarate in a dosage of about 10 mg/day to about 50 mg/day. In some embodiments, the administration is performed one to three times between about 72 hours to about 1 hour before exposure of the subject to the HIV and/or one to three times about 1 hour to about 72 hours after exposure of the subject to the HIV. In some embodiments, the administration is performed one to three times about 1 hour to about 72 hours after exposure of the subject to the HIV.

The present invention further provides a method of preventing an HIV infection in a subject, comprising administering to the subject:
(a) bictegravir, or a pharmaceutically acceptable salt thereof, in a dosage of about 10 mg/day to about 200 mg/day;
(b) emtricitabine, or a pharmaceutically acceptable salt thereof, in a dosage of about 100 mg/day to about 400 mg/day; and
(c) tenofovir disoproxil, or a pharmaceutically acceptable salt thereof, in a dosage of about 10 mg/day to about 500 mg/day. In some embodiments, the administration is performed one to three times between about 72 hours to about 1 hour before exposure of the subject to the HIV and/or one to three times about 1 hour to about 72 hours after exposure of the subject to the HIV. In some embodiments, the administration is performed one to three times about 1 hour to about 72 hours after exposure of the subject to the HIV.

The present invention further provides a method of preventing an HIV infection in a subject, comprising administering to the subject:
(a) bictegravir, or a pharmaceutically acceptable salt thereof, in a dosage of about 10 mg/day to about 200 mg/day;
(b) emtricitabine, or a pharmaceutically acceptable salt thereof, in a dosage of about 100 mg/day to about 400 mg/day; and
(c) tenofovir disoproxil, or a pharmaceutically acceptable salt thereof, in a dosage of about 10 mg/day to about 500 mg/day. In some embodiments, the administration is performed one to three times between about 72 hours to about 1 hour before exposure of the subject to the HIV and/or one to three times about 1 hour to about 72 hours after exposure of the subject to the HIV. In some embodiments, the administration is performed one to three times about 1 hour to about 72 hours after exposure of the subject to the HIV.

The present invention further provides a method of preventing an HIV infection in a subject, comprising administering to the subject:
(a) bictegravir sodium in a dosage of about 10 mg/day to about 200 mg/day;
(b) emtricitabine in a dosage of about 100 mg/day to about 400 mg/day; and
(c) tenofovir disoproxil fumarate in a dosage of about 10 mg/day to about 500 mg/day. In some embodiments, the administration is performed one to three times between about 72 hours to about 1 hour before exposure of the subject to the HIV and/or one to three times about 1 hour to about 72 hours after exposure of the subject to the HIV. In some embodiments, the administration is performed one to three times about 1 hour to about 72 hours after exposure of the subject to the HIV.

The present invention further provides a method of preventing an HIV infection in a subject, comprising administering to the subject:
(a) bictegravir sodium in a dosage of about 10 mg/day to about 200 mg/day;
(b) emtricitabine in a dosage of about 100 mg/day to about 400 mg/day; and
(c) tenofovir disoproxil fumarate in a dosage of about 10 mg/day to about 500 mg/day. In some embodiments, the administration is performed one to three times between about 72 hours to about 1 hour before exposure of the subject to the HIV and/or one to three times about 1 hour to about 72 hours after exposure of the subject to the HIV. In some embodiments, the administration is performed one to three times about 1 hour to about 72 hours after exposure of the subject to the HIV.

In some embodiments, the present invention further provides methods for reducing the risk of acquiring HIV (*e.g.*, HIV-1 and/or HIV-2), comprising administering to the subject bictegravir, or a pharmaceutically acceptable salt thereof, in combination with one or more additional therapeutic agents as described herein. For example, methods for reducing the risk of acquiring HIV (*e.g.*, HIV-1 and/or HIV-2) comprise administration of bictegravir, or a pharmaceutically acceptable salt thereof, in combination with one, two, or three additional therapeutic agents as disclosed herein. In some embodiments, the administration is performed one to three times about 1 hour to about 72 hours after exposure of the subject to the HIV.

In some embodiments, the present invention provides a method of reducing the risk of acquiring HIV in a subject, comprising administering to the subject:
(a) bictegravir, or a pharmaceutically acceptable salt thereof, in a dosage of about 10 mg/day to about 200 mg/day;
(b) emtricitabine, or a pharmaceutically acceptable salt thereof, in a dosage of about 100 mg/day to about 400 mg/day; and
(c) tenofovir alafenamide, or a pharmaceutically acceptable salt thereof, in a dosage of about 10 mg/day to about 50 mg/day. In some embodiments, the administration is performed one to three times between about 72 hours to about 1 hour before exposure of the subject to the HIV and/or one to three times about 1 hour to about 72 hours after exposure of the subject to the HIV. In some embodiments, the administration is performed one to three times about 1 hour to about 72 hours after exposure of the subject to the HIV.

In some embodiments, the present invention provides a method of reducing the risk of acquiring HIV in a subject, comprising administering to the subject:
(a) bictegravir sodium in a dosage of about 10 mg/day to about 200 mg/day;
(b) emtricitabine in a dosage of about 100 mg/day to about 400 mg/day; and
(c) tenofovir alafenamide hemifumarate in a dosage of about 10 mg/day to about 50 mg/day. In some embodiments, the administration is performed one to three times between about 72 hours to about 1 hour before exposure of the subject to the HIV and/or one to three times about 1 hour to about 72 hours after exposure of the subject to the HIV. In some embodiments, the administration is performed one to three times about 1 hour to about 72 hours after exposure of the subject to the HIV.

In some embodiments, the present invention provides a method of reducing the risk of acquiring HIV in a subject, comprising administering to the subject:
(a) bictegravir, or a pharmaceutically acceptable salt thereof, in a dosage of about 10 mg/day to about 200 mg/day;
(b) emtricitabine, or a pharmaceutically acceptable salt thereof, in a dosage of about 100 mg/day to about 400 mg/day; and
(c) tenofovir disoproxil, or a pharmaceutically acceptable salt thereof, in a dosage of about 10 mg/day to about 500 mg/day. In some embodiments, the administration is performed one to three times between about 72 hours to about 1 hour before exposure of the subject to the HIV and/or one to three times about 1 hour to about 72 hours after exposure of the subject to the HIV. In some embodiments, the administration is performed one to three times about 1 hour to about 72 hours after exposure of the subject to the HIV.

In some embodiments, the present invention provides a method of reducing the risk of acquiring HIV in a subject, comprising administering to the subject:
(a) bictegravir, or a pharmaceutically acceptable salt thereof, in a dosage of about 10 mg/day to about 200 mg/day;
(b) emtricitabine, or a pharmaceutically acceptable salt thereof, in a dosage of about 100 mg/day to about 400 mg/day; and
(c) tenofovir disoproxil, or a pharmaceutically acceptable salt thereof, in a dosage of about 10 mg/day to about 500 mg/day. In some embodiments, the administration is performed one to three times between about 72 hours to about 1 hour before exposure of the subject to the HIV and/or one to three times about 1 hour to about 72 hours after exposure of the subject to the HIV. In some embodiments, the administration is performed one to three times about 1 hour to about 72 hours after exposure of the subject to the HIV.

In some embodiments, the present invention provides a method of reducing the risk of acquiring HIV in a subject, comprising administering to the subject:
(a) bictegravir sodium in a dosage of about 10 mg/day to about 200 mg/day;
(b) emtricitabine in a dosage of about 100 mg/day to about 400 mg/day; and
(c) tenofovir disoproxil fumarate in a dosage of about 10 mg/day to about 500 mg/day. In some embodiments, the administration is performed one to three times between about 72 hours to about 1 hour before exposure of the subject to the HIV and/or one to three times about 1 hour to about 72 hours after exposure of the subject to the HIV. In some embodiments, the administration is performed one to three times about 1 hour to about 72 hours after exposure of the subject to the HIV.

In some embodiments, the present invention provides a method of reducing the risk of acquiring HIV in a subject, comprising administering to the subject:
(a) bictegravir sodium in a dosage of about 10 mg/day to about 200 mg/day;
(b) emtricitabine in a dosage of about 100 mg/day to about 400 mg/day; and
(c) tenofovir disoproxil fumarate in a dosage of about 10 mg/day to about 500 mg/day. In some embodiments, the administration is performed one to three times between about 72 hours to about 1 hour before exposure of the subject to the HIV and/or one to three times about 1 hour to about 72 hours after exposure of the subject to the HIV. In some embodiments, the administration is performed one to three times about 1 hour to about 72 hours after exposure of the subject to the HIV.

The present invention further provides a method of preventing an HIV infection in a subject, comprising administering to the subject:
(a) bictegravir, or a pharmaceutically acceptable salt thereof, in a dosage of about 10 mg/day to about 100 mg/day;
(b) emtricitabine, or a pharmaceutically acceptable salt thereof, in a dosage of about 100 mg/day to about 300 mg/day; and
(c) tenofovir alafenamide, or a pharmaceutically acceptable salt thereof, in a dosage of about 10 mg/day to about 50 mg/day. In some embodiments, the administration is performed about 72 hours to about 1 hour before exposure of the subject to the HIV or about 1 hour to about 72 hours after exposure of the subject to the HIV.

The present invention further provides a method of preventing an HIV infection in a subject, comprising administering to the subject:
(a) bictegravir sodium, in a dosage of about 10 mg/day to about 100 mg/day;
(b) emtricitabine in a dosage of about 100 mg/day to about 300 mg/day; and
(c) tenofovir alafenamide hemifumarate in a dosage of about 10 mg/day to about 50 mg/day. In some embodiments, the administration is performed about 72 hours to about 1 hour before exposure of the subject to the HIV or about 1 hour to about 72 hours after exposure of the subject to the HIV.

The present invention further provides a method of preventing an HIV infection in a subject, comprising administering to the subject:
(a) bictegravir, or a pharmaceutically acceptable salt thereof, in a dosage of about 10 mg/day to about 100 mg/day;
(b) emtricitabine, or a pharmaceutically acceptable salt thereof, in a dosage of about 100 mg/day to about 300 mg/day; and
(c) tenofovir disoproxil, or a pharmaceutically acceptable salt thereof, in a dosage of about 10 mg/day to about 500 mg/day. In some embodiments, the administration is performed about 72 hours to about 1 hour before exposure of the subject to the HIV or about 1 hour to about 72 hours after exposure of the subject to the HIV.

The present invention further provides a method of preventing an HIV infection in a subject, comprising administering to the subject:
(a) bictegravir, or a pharmaceutically acceptable salt thereof, in a dosage of about 10 mg/day to about 100 mg/day;
(b) emtricitabine, or a pharmaceutically acceptable salt thereof, in a dosage of about 100 mg/day to about 300 mg/day; and
(c) tenofovir disoproxil, or a pharmaceutically acceptable salt thereof, in a dosage of about 10 mg/day to about 500 mg/day. In some embodiments, the administration is performed about 72 hours to about 1 hour before exposure of the subject to the HIV or about 1 hour to about 72 hours after exposure of the subject to the HIV.

The present invention further provides a method of preventing an HIV infection in a subject, comprising administering to the subject:
(a) bictegravir sodium in a dosage of about 10 mg/day to about 100 mg/day;
(b) emtricitabine in a dosage of about 100 mg/day to about 300 mg/day; and
(c) tenofovir disoproxil fumarate in a dosage of about 10 mg/day to about 500 mg/day. In some embodiments, the administration is performed about 72 hours to about 1 hour before exposure of the subject to the HIV or about 1 hour to about 72 hours after exposure of the subject to the HIV.

The present invention further provides a method of preventing an HIV infection in a subject, comprising administering to the subject:
(a) bictegravir sodium in a dosage of about 10 mg/day to about 100 mg/day;
(b) emtricitabine in a dosage of about 100 mg/day to about 300 mg/day; and
(c) tenofovir disoproxil fumarate in a dosage of about 10 mg/day to about 500 mg/day. In some embodiments, the administration is performed about 72 hours to about 1 hour before exposure of the subject to the HIV or about 1 hour to about 72 hours after exposure of the subject to the HIV.

In some embodiments, the present invention further provides methods for reducing the risk of acquiring HIV (e.g., HIV-1 and/or HIV-2), comprising administering to the subject bictegravir, or a pharmaceutically acceptable salt thereof, in combination with one or more additional therapeutic agents as described herein. For example, methods for reducing the risk of acquiring HIV (e.g., HIV-1 and/or HIV-2) comprise administration of bictegravir, or a pharmaceutically acceptable salt thereof, in combination with one, two, or three additional therapeutic agents as disclosed herein.

In some embodiments, the present invention provides a method of reducing the risk of acquiring HIV in a subject, comprising administering to the subject:
(a) bictegravir, or a pharmaceutically acceptable salt thereof, in a dosage of about 10 mg/day to about 100 mg/day;
(b) emtricitabine, or a pharmaceutically acceptable salt thereof, in a dosage of about 100 mg/day to about 300 mg/day; and
(c) tenofovir alafenamide, or a pharmaceutically acceptable salt thereof, in a dosage of about 10 mg/day to about 50 mg/day. In some embodiments, the administration is performed about 72 hours to about 1 hour before exposure of the subject to the HIV or about 1 hour to about 72 hours after exposure of the subject to the HIV.

In some embodiments, the present invention provides a method of reducing the risk of acquiring HIV in a subject, comprising administering to the subject:
(a) bictegravir sodium in a dosage of about 10 mg/day to about 100 mg/day;
(b) emtricitabine in a dosage of about 100 mg/day to about 300 mg/day; and
(c) tenofovir alafenamide hemifumarate in a dosage of about 10 mg/day to about 50 mg/day. In some embodiments, the administration is performed about 72 hours to about 1 hour before exposure of the subject to the HIV or about 1 hour to about 72 hours after exposure of the subject to the HIV.

In some embodiments, the present invention provides a method of reducing the risk of acquiring HIV in a subject, comprising administering to the subject:
(a) bictegravir, or a pharmaceutically acceptable salt thereof, in a dosage of about 10 mg/day to about 100 mg/day;
(b) emtricitabine, or a pharmaceutically acceptable salt thereof, in a dosage of about 100 mg/day to about 300 mg/day; and
(c) tenofovir disoproxil, or a pharmaceutically acceptable salt thereof, in a dosage of about 10 mg/day to about 500 mg/day. In some embodiments, the administration is performed about 72 hours to about 1 hour before exposure of the subject to the HIV or about 1 hour to about 72 hours after exposure of the subject to the HIV.

In some embodiments, the present invention provides a method of reducing the risk of acquiring HIV in a subject, comprising administering to the subject:
(a) bictegravir, or a pharmaceutically acceptable salt thereof, in a dosage of about 10 mg/day to about 100 mg/day;
(b) emtricitabine, or a pharmaceutically acceptable salt thereof, in a dosage of about 100 mg/day to about 300 mg/day; and
(c) tenofovir disoproxil, or a pharmaceutically acceptable salt thereof, in a dosage of about 10 mg/day to about 500 mg/day. In some embodiments, the administration is performed about 72 hours to about 1 hour before exposure of the subject to the HIV or about 1 hour to about 72 hours after exposure of the subject to the HIV.

In some embodiments, the present invention provides a method of reducing the risk of acquiring HIV in a subject, comprising administering to the subject:
(a) bictegravir sodium in a dosage of about 10 mg/day to about 100 mg/day;
(b) emtricitabine in a dosage of about 100 mg/day to about 300 mg/day; and
(c) tenofovir disoproxil fumarate in a dosage of about 10 mg/day to about 500 mg/day. In some embodiments, the administration is performed about 72 hours to about 1 hour before exposure of the subject to the HIV or about 1 hour to about 72 hours after exposure of the subject to the HIV.

In some embodiments, the present invention provides a method of reducing the risk of acquiring HIV in a subject, comprising administering to the subject:
(a) bictegravir sodium in a dosage of about 10 mg/day to about 100 mg/day;
(b) emtricitabine in a dosage of about 100 mg/day to about 300 mg/day; and
(c) tenofovir disoproxil fumarate in a dosage of about 10 mg/day to about 500 mg/day. In some embodiments, the administration is performed about 72 hours to about 1 hour before exposure of the subject to the HIV or about 1 hour to about 72 hours after exposure of the subject to the HIV.

In certain embodiments, the methods described herein comprise administration of:
(a) bictegravir as a pharmaceutically acceptable salt in a dosage of about 100 mg/day;
(b) emtricitabine in a dosage of about 400 mg/day; and
(c) tenofovir alafenamide as a pharmaceutically acceptable salt in a dosage of about 50 mg/day.

In certain embodiments, the methods described herein comprise administration of:
(a) bictegravir as a pharmaceutically acceptable salt in a dosage of about 100 mg/day;
(b) emtricitabine in a dosage of about 200 mg/day; and
(c) tenofovir alafenamide as a pharmaceutically acceptable salt in a dosage of about 25 mg/day.

In certain embodiments, the methods described herein comprise administration of:
(a) bictegravir as a pharmaceutically acceptable salt in a dosage of about 50 mg/day;
(b) emtricitabine in a dosage of about 200 mg/day; and
(c) tenofovir alafenamide as a pharmaceutically acceptable salt in a dosage of about 25 mg/day.

In certain embodiments, the methods described herein comprise administration of:
(a) bictegravir as a pharmaceutically acceptable salt in a dosage of about 25 mg/day;
(b) emtricitabine in a dosage of about 200 mg/day; and
(c) tenofovir disoproxil as a pharmaceutically acceptable salt in a dosage of about 245 mg/day.

In certain embodiments, the methods described herein comprise administration of:
(a) bictegravir as a pharmaceutically acceptable salt in a dosage of about 25 mg/day;
(b) emtricitabine in a dosage of about 200 mg/day; and
(c) tenofovir disoproxil as a pharmaceutically acceptable salt in a dosage of about 300 mg/day.

In certain embodiments, the methods described herein comprise administration of:
(a) bictegravir as a pharmaceutically acceptable salt in a dosage of about 25 mg/day;
(b) emtricitabine in a dosage of about 200 mg/day; and
(c) tenofovir disoproxil fumarate in a dosage of about 300 mg/day.

In certain embodiments, the methods described herein comprise administration of:
(a) bictegravir sodium in a dosage of about 104 mg/day;
(b) emtricitabine in a dosage of about 400 mg/day; and
(c) tenofovir alafenamide hemifumarate in a dosage of about 56 mg/day.

In certain embodiments, the methods described herein comprise administration of:
(a) bictegravir sodium in a dosage of about 104 mg/day;
(b) emtricitabine in a dosage of about 200 mg/day; and
(c) tenofovir alafenamide hemifumarate in a dosage of about 28 mg/day.

In certain embodiments, the methods described herein comprise administration of:
(a) bictegravir sodium in a dosage of about 52 mg/day;
(b) emtricitabine in a dosage of about 200 mg/day; and
(c) tenofovir alafenamide hemifumarate in a dosage of about 28 mg/day.

The present invention further provides a method of preventing an HIV infection in a subject, comprising administering to the subject:
(a) elvitegravir, or a pharmaceutically acceptable salt thereof, in a dosage of about 100 mg/day to about 200 mg/day;
(b) emtricitabine, or a pharmaceutically acceptable salt thereof, in a dosage of about 100 mg/day to about 300 mg/day; and
(c) tenofovir alafenamide, or a pharmaceutically acceptable salt thereof, in a dosage of about 10 mg/day to about 50 mg/day. In some embodiments, the administration is performed about 72 hours to about 1 hour before exposure of the subject to the HIV or about 1 hour to about 72 hours after exposure of the subject to the HIV.

The present invention further provides a method of preventing an HIV infection in a subject, comprising administering to the subject:
(a) elvitegravir, or a pharmaceutically acceptable salt thereof, in a dosage of about 100 mg/day to about 200 mg/day;
(b) emtricitabine in a dosage of about 100 mg/day to about 300 mg/day; and
(c) tenofovir alafenamide hemifumarate in a dosage of about 10 mg/day to about 50 mg/day. In some embodiments, the administration is performed about 72 hours to about 1 hour before exposure of the subject to the HIV or about 1 hour to about 72 hours after exposure of the subject to the HIV.

The present invention further provides a method of preventing an HIV infection in a subject, comprising administering to the subject:
(a) elvitegravir, or a pharmaceutically acceptable salt thereof, in a dosage of about 100 mg/day to about 200 mg/day;
(b) emtricitabine, or a pharmaceutically acceptable salt thereof, in a dosage of about 100 mg/day to about 300 mg/day; and
(c) tenofovir disoproxil, or a pharmaceutically acceptable salt thereof, in a dosage of about 10 mg/day to about 500 mg/day. In some embodiments, the administration is performed about 72 hours to about 1 hour before exposure of the subject to the HIV or about 1 hour to about 72 hours after exposure of the subject to the HIV.

The present invention further provides a method of preventing an HIV infection in a subject, comprising administering to the subject:
(a) elvitegravir, or a pharmaceutically acceptable salt thereof, in a dosage of about 100 mg/day to about 200 mg/day;
(b) emtricitabine, or a pharmaceutically acceptable salt thereof, in a dosage of about 100 mg/day to about 300 mg/day; and
(c) tenofovir disoproxil, or a pharmaceutically acceptable salt thereof, in a dosage of about 10 mg/day to about 500 mg/day. In some embodiments, the administration is performed about 72 hours to about 1 hour before exposure of the subject to the HIV or about 1 hour to about 72 hours after exposure of the subject to the HIV.

The present invention further provides a method of preventing an HIV infection in a subject, comprising administering to the subject:
(a) elvitegravir, or a pharmaceutically acceptable salt thereof, in a dosage of about 100 mg/day to about 200 mg/day;
(b) emtricitabine in a dosage of about 100 mg/day to about 300 mg/day; and
(c) tenofovir disoproxil fumarate in a dosage of about 10 mg/day to about 500 mg/day. In some embodiments, the administration is performed about 72 hours to about 1 hour before exposure of the subject to the HIV or about 1 hour to about 72 hours after exposure of the subject to the HIV.

The present invention further provides a method of preventing an HIV infection in a subject, comprising administering to the subject:
(a) elvitegravir, or a pharmaceutically acceptable salt thereof, in a dosage of about 100 mg/day to about 200 mg/day;
(b) emtricitabine in a dosage of about 100 mg/day to about 300 mg/day; and
(c) tenofovir disoproxil fumarate in a dosage of about 10 mg/day to about 500 mg/day. In some embodiments, the administration is performed about 72 hours to about 1 hour before exposure of the subject to the HIV or about 1 hour to about 72 hours after exposure of the subject to the HIV.

In some embodiments, the present invention provides a method of reducing the risk of acquiring HIV in a subject, comprising administering to the subject:
(a) elvitegravir, or a pharmaceutically acceptable salt thereof, in a dosage of about 100 mg/day to about 200 mg/day;
(b) emtricitabine, or a pharmaceutically acceptable salt thereof, in a dosage of about 100 mg/day to about 300 mg/day; and
(c) tenofovir alafenamide, or a pharmaceutically acceptable salt thereof, in a dosage of about 10 mg/day to about 50 mg/day. In some embodiments, the administration is performed about 72 hours to about 1 hour before exposure of the subject to the HIV or about 1 hour to about 72 hours after exposure of the subject to the HIV.

In some embodiments, the present invention provides a method of reducing the risk of acquiring HIV in a subject, comprising administering to the subject:
(a) elvitegravir, or a pharmaceutically acceptable salt thereof, in a dosage of about 100 mg/day to about 200 mg/day;
(b) emtricitabine in a dosage of about 100 mg/day to about 300 mg/day; and
(c) tenofovir alafenamide hemifumarate in a dosage of about 10 mg/day to about 50 mg/day. In some embodiments, the administration is performed about 72 hours to about 1 hour before exposure of the subject to the HIV or about 1 hour to about 72 hours after exposure of the subject to the HIV.

In some embodiments, the present invention provides a method of reducing the risk of acquiring HIV in a subject, comprising administering to the subject:
(a) elvitegravir, or a pharmaceutically acceptable salt thereof, in a dosage of about 100 mg/day to about 200 mg/day;
(b) emtricitabine, or a pharmaceutically acceptable salt thereof, in a dosage of about 100 mg/day to about 300 mg/day; and
(c) tenofovir disoproxil, or a pharmaceutically acceptable salt thereof, in a dosage of about 10 mg/day to about 500 mg/day. In some embodiments, the administration is performed about 72 hours to about 1 hour before exposure of the subject to the HIV or about 1 hour to about 72 hours after exposure of the subject to the HIV.

In some embodiments, the present invention provides a method of reducing the risk of acquiring HIV in a subject, comprising administering to the subject:
(a) elvitegravir, or a pharmaceutically acceptable salt thereof, in a dosage of about 100 mg/day to about 200 mg/day;
(b) emtricitabine, or a pharmaceutically acceptable salt thereof, in a dosage of about 100 mg/day to about 300 mg/day; and
(c) tenofovir disoproxil, or a pharmaceutically acceptable salt thereof, in a dosage of about 10 mg/day to about 500 mg/day. In some embodiments, the administration is performed about 72 hours to about 1 hour before exposure of the subject to the HIV or about 1 hour to about 72 hours after exposure of the subject to the HIV.

In some embodiments, the present invention provides a method of reducing the risk of acquiring HIV in a subject, comprising administering to the subject:
(a) elvitegravir, or a pharmaceutically acceptable salt thereof, in a dosage of about 100 mg/day to about 200 mg/day;
(b) emtricitabine in a dosage of about 100 mg/day to about 300 mg/day; and
(c) tenofovir disoproxil fumarate in a dosage of about 10 mg/day to about 500 mg/day. In some embodiments, the administration is performed about 72 hours to about 1 hour before exposure of the subject to the HIV or about 1 hour to about 72 hours after exposure of the subject to the HIV.

In some embodiments, the present invention provides a method of reducing the risk of acquiring HIV in a subject, comprising administering to the subject:
(a) elvitegravir, or a pharmaceutically acceptable salt thereof, in a dosage of about 100 mg/day to about 200 mg/day;
(b) emtricitabine in a dosage of about 100 mg/day to about 300 mg/day; and
(c) tenofovir disoproxil fumarate in a dosage of about 10 mg/day to about 500 mg/day. In some embodiments, the administration is performed about 72 hours to about 1 hour before exposure of the subject to the HIV or about 1 hour to about 72 hours after exposure of the subject to the HIV.

In certain embodiments, the methods described herein comprise administration of:
(a) elvitegravir as a pharmaceutically acceptable salt in a dosage of about 50 mg/day;
(b) emtricitabine in a dosage of about 200 mg/day; and
(c) tenofovir alafenamide as a pharmaceutically acceptable salt in a dosage of about 25 mg/day.

In certain embodiments, the methods described herein comprise administration of:
(a) elvitegravir as a pharmaceutically acceptable salt in a dosage of about 25 mg/day;
(b) emtricitabine in a dosage of about 200 mg/day; and
(c) tenofovir disoproxil as a pharmaceutically acceptable salt in a dosage of about 245 mg/day.

In certain embodiments, the methods described herein comprise administration of:
(a) elvitegravir as a pharmaceutically acceptable salt in a dosage of about 25 mg/day;
(b) emtricitabine in a dosage of about 200 mg/day; and
(c) tenofovir disoproxil as a pharmaceutically acceptable salt in a dosage of about 300 mg/day.

In certain embodiments, the methods described herein comprise administration of:
(a) elvitegravir as a pharmaceutically acceptable salt in a dosage of about 25 mg/day;
(b) emtricitabine in a dosage of about 200 mg/day; and
(c) tenofovir disoproxil fumarate in a dosage of about 300 mg/day.

In certain embodiments, the methods described herein comprise administration of:
(a) elvitegravir sodium in a dosage of about 52 mg/day;
(b) emtricitabine in a dosage of about 200 mg/day; and
(c) tenofovir alafenamide hemifumarate in a dosage of about 28 mg/day.

In certain embodiments, the methods described further comprise administration of cobicistat, or a pharmaceutically acceptable salt thereof. In certain embodiments, the methods described further comprise administration of cobicistat, or a pharmaceutically acceptable salt thereof, in a dosage of about 100 mg/day to about 300 mg/day. In certain embodiments, the methods described further comprise administration of cobicistat in a dosage of about 200 mg/day.

In certain embodiments, the reduction in risk of acquiring HIV is at least about 40%, 50%, 60%, 70%, 80%, 90%, or 95%. In certain embodiments, the reduction in risk of acquiring HIV is at least about 75%. In certain embodiments, the reduction in risk of acquiring HIV is about 80%, 85%, or 90%.

In certain embodiments, when the compound of Formula (I) or Formula (II), or a pharmaceutically acceptable salt thereof, is combined with one or more additional therapeutic agents as described above, the components of the composition are administered as a simultaneous or sequential regimen. When administered sequentially, the combination may be administered in two or more administrations.

In certain embodiments, the compound of Formula (I) or Formula (II) is combined with one or more additional therapeutic agents in a unitary dosage form for simultaneous administration to a subject, for example as a solid dosage form for oral administration (*e.g.*, a fixed dose combination tablet).

Co-administration of the compound of Formula (I) or Formula (II), or a pharmaceutically acceptable salt thereof, with one or more additional therapeutic agents generally refers to simultaneous or sequential administration of the compound of Formula (I) or Formula (II) and one or more additional therapeutic agents, such that therapeutically effective amounts of the compound of Formula (I) or Formula (II) and one or more additional therapeutic agents are both present in the body of the subject.

Co-administration includes administration of unit dosages of bictegravir, or a pharmaceutically acceptable salt thereof, before or after administration of unit dosages of one or more additional therapeutic agents, for example, administration of the compound of Formula (I) or Formula (II) or salt thereof within seconds, minutes, or hours of the administration of one or more additional therapeutic agents. For example, in some embodiments, a unit dose of the compound of Formula (I) or Formula (II), or a pharmaceutically acceptable salt thereof, is administered first, followed within seconds or minutes by administration of a unit dose of one or more additional therapeutic agents. Alternatively, in other embodiments, a unit dose of one or more additional therapeutic agents is administered first, followed by administration of a unit dose of the compound of Formula (I) or Formula (II), or a pharmaceutically acceptable salt thereof, within seconds or minutes. In some embodiments, a unit dose of the compound of Formula (I) or Formula (II), or a pharmaceutically acceptable salt thereof, is administered first, followed, after a period of hours (*e*.*g*., 1-12 hours), by administration of a unit dose of one or more additional therapeutic agents. In other embodiments, a unit dose of one or more additional therapeutic agents is administered first, followed, after a period of hours (*e*.*g*., 1-12 hours), by administration of a unit dose of the compound of Formula (I) or Formula (II), or a pharmaceutically acceptable salt thereof.

The invention will be described in greater detail by way of specific examples. The following examples are offered for illustrative purposes, and are not intended to limit the invention in any manner. Those of skill in the art will readily recognize a variety of noncritical parameters which can be changed or modified to yield essentially the same results.

### EXAMPLES

### Example 1. Three-arm, two-stage HIV prophylactic vaccine trial with a second randomisation to evaluate the proportion of HIV infections averted by TAF/FTC in comparison to TDF/FTC PrEP

The title example will be performed according to the following protocol:
Design: Phase III, three-arm randomisation comparing each of 2 experimental combination vaccine regimens with placebo control. There will be a second 1:1 randomisation comparing two PrEP regimens, open-label daily TDF/FTC or TAF/FTC. Subjects will receive study PrEP through week 26 after which access to PrEP will revert to local supply. Pre-screening for HIV status will take place as part of a Registration Cohort which will precede and continue in parallel to PrEP vaccination enrollments.
Objectives/aims: 1) Measure HIV incidence during the first 26 weeks (PrEP period); 2) Measure HIV incidence from week 26 to week 74 among subjects that receive all 3 immunizations; 3) Measure adverse events leading to a clinical decision to discontinue PrEP.
Duration: 40 weeks of PrEP per subject (during the immunization period).

The trial can further be modified to replace TAF/FTC with BIC or EVG optionally in combination with FTC and TAF.

### Example 2. Evaluation of pre-exposure prophylaxis (PrEP) initiation, retention and adherence in pregnant and breastfeeding women

The title example will be performed according to the following protocol:
Design: Observational cohort study in 1200 pregnant women who will be recruited at the first antenatal care (ANC) visit (n=600 pregnant women per site).
Objectives/aims: 1) Determine the distribution of women across the PrEP cascade (initiation, retention, and adherence) and outcomes (HIV acquisition, transmission, and adverse events) in a cohort of pregnant and breastfeeding women using quantitative and qualitative approaches; 2) Evaluate subject and provider-level factors associated with the PrEP cascade using quantitative and qualitative approaches; 3) Apply an established mathematical model to simulate the impact of improvement in the PrEP cascade on HIV infections averted (maternal and perinatal).
Sample size: 1200
Duration: 18 months average

### Example 3. Single-arm longitudinal study to offer daily FTC/TDF as PrEP for Periconception Use to HIV-Uninfected Women

The title example is performed according to the following protocol:
Design: Single-arm longitudinal study to offer daily FTC/TDF as PrEP for periconception use to HIV-uninfected women who report plans for pregnancy with an infected or unknown serostatus partner. Women will have the option to take PrEP during pregnancy. PrEP will be offered as part of a safer conception package inclusive of couples-based HIV counselling and testing (CHCT), antiretroviral therapy (ART) for the infected partner, treatment for STIs and safer conception strategies, such as, limiting sex without condoms to peak fertility.
Objectives/aims: 1) Measure uptake of PrEP by women, during periconception and pregnancy follow up; 2) Measure adherence to PrEP using who initiate periconception PrEP and continue PrEP during pregnancy (quarterly plasma concentrations, daily electronic pill cap); 3) Use qualitative methods to explore PrEP adherence promoters and barriers during periconception and pregnancy to inform conceptual framework.
Sample size: 350 HIV-uninfected women; 67 subject enrolled as of August 2018.
Duration: Minimum of 12 months (women not currently pregnant) and a maximum of 24 months (women who become pregnant during the first 12 months).

This study can be modified to replace FTC/TDF with BIC or EVG optionally in combination with FTC and TAF (or TDF).

### Example 4. Immediate or Deferred PrEP for HIV Prevention

The title example is performed according to the following protocol:
Design: Open-label randomized controlled study/
Primary Objective: Compare the frequency and seriousness of adverse events in women and their infants in Arm A against Arm B where Arm A is an allocation to immediate PrEP in pregnancy and Arm B is to have PrEP deferred until after delivery.
Sample size: 842 (1:1); 268 subjects enrolled as of August 2018.

The title trial commenced in October 2017 and is ongoing with anticipated 400 subject visits (repeat study visits) per month.

### Example 5. Evaluation of Genvoya^{®} for Event-Driven PrEP/PEP in Non-Human Primate Model

This study will be performed to establish a minimal effective dosing regimen of Genvoya^{®} (cobicistat/elvitegravir/emtricitabine/tenofovir) for HIV PrEP/PEP "on demand" using a non-human primate (NHP) animal model. PEP regimen is initiated as soon as possible ≤72 hours from exposure. Two study designs are shown below in Table 1.

**Table 1.**

| **Indication** & **Usage** | Pre-exposure prophylaxis (PrEP) | PrEP or Post-exposure prophylaxis (PEP) |
|---|---|---|
| **Target Population** | HIV-negative adults aged 18 years or older males and females | HIV-negative adolescents aged 12 years or older males and females, transgender |
| **Efficacy & Safety** | Same Contraindication/ Warnings and Precautions as GEN label or comparable to oral ARVs | Same Contraindication/ Warnings and Precautions as GEN label or comparable to oral ARVs |
| **Frequency & Dosing** | 1. One single dose 2-24 hrs before having sex and one single dose 24 hrs after | 1. One single dose 2-24 hrs before sex and one single dose 24 hrs after, or 2 single doses within 24 hrs or 48 hrs (2-dose PrEP) |
| | *planned event driven (short course)* | 2. One single dose 2-24 hrs before sex and one single dose 24 hrs after, or 2-7 single doses within 48/72 hrs |
| | | *unplanned event driven (short course)* |
| **Other Attributes** | Specify median # of X sex acts per mo. and/or X partners every two mo. | |

Rhesus macaques of Indian origin are the best characterized and most utilized non-human primate model for HIV transmission (see *e*.*g*., Hatziioannou and Evans. Nature Rev Microbiol, 2012). Infection of these animals with SIV recapitulates hallmarks of HIV-1 pathogenesis (Del Prete and Lifson. Curr Top Microbiol Immunol, 2017). SHIV is a chimeric virus bearing R5 tropic HIV-1 envelope, which readily infects macaques and resembles naturally transmitted virus in the human population.

The combination of FTC with TDF or TAF is highly effective at preventing rectal infection with SHIV in rhesus macaques (see *e*.*g*., recent published data summarized in Table 2). More recently, this work was extended to the prevention of vaginal viral challenge with SHIV in a pigtail macaque model. Efforts are currently underway to identify short and potent regimens with GEN (E/C/F/TAF) in rhesus macaques. Without being bound by theory, an addition of an INSTI, a drug class which targets a later step in the viral life cycle relative to RT inhibitors, is hypothesized to further improve protection.

**Table 2.**

| NA = data not available | | | | | | | | | | |
|---|---|---|---|---|---|---|---|---|---|---|
| Garcia-Lerma et al. Plos Med, 2008 | Rectal | FTC+TDF (SQ) | | -2h | | +24h | | 14 | 6/6 | 1/18 |
| Garcia-Lerma et al. Sci Trans Med, 2010 | Rectal | FTC+TDF (SQ & PO) | | -2h | | | | 14 | 4/6 | 0/9 |
| | | | -22h | | +2h | | | | 5/6 | |
| | | | | -2h | | +22h | | | 3/6 | |
| | | | | | +2h | +26h | | | 2/4 | |
| Massud, et al. JID, 2016 | Rectal | FTC+TAF (PO) | -24h | | +2h | | | 19 | 6/6 | 0/6 |
| Massud, et al. CROI abstract 2018 | Vaginal | FTC+TAF (PO) | -24h | | +2h | | | 16 | 5/6 | 0/5 |

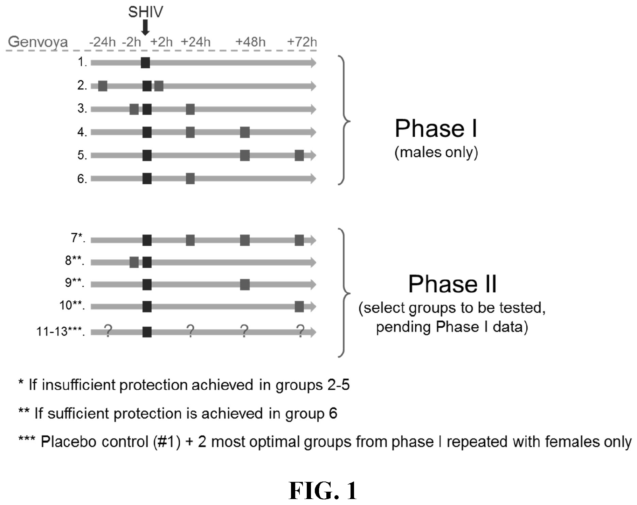

The present study is summarized in Table 3 and Figure 1. Genvoya will be dosed by oral gavage to anesthetized animals as detailed in the study groups schema (FIG. 1). Plasma viral loads will be measured by standard qPCR assay at 2-week intervals to confirm infection. The animals will be monitored for a total of at least 60 days following the last challenge. The resulting rates of protection relative to placebo will determine the minimal effective dosing regimen.

**Table 3.**

| Species | Indian Rhesus Macaques (even split of males and females) |
|---|---|
| N per group | N= 6 (+/-1) |
| Inoculation route | Rectal |
| Virus strain | SHIV 162P3 |
| Total exposures / animal | Single exposure EOW, n=6 total |
| Virus dose | 10-50 TCID₅₀ |
| Route of drug administration | PO. Genvoya pills crushed and dosed in PBS vs placebo. Dose to match human dose. |

The proposed treatment groups are summarized in Figure 1. These may be split into multiple studies and carried out sequentially. This study can be modified to replace Genvoya^{®} with BIC, optionally in combination with FTC and TAF (or TDF).

### Example 6. Evaluation of BIC/FTC/TAF for PEP in Non-Human Primate Model

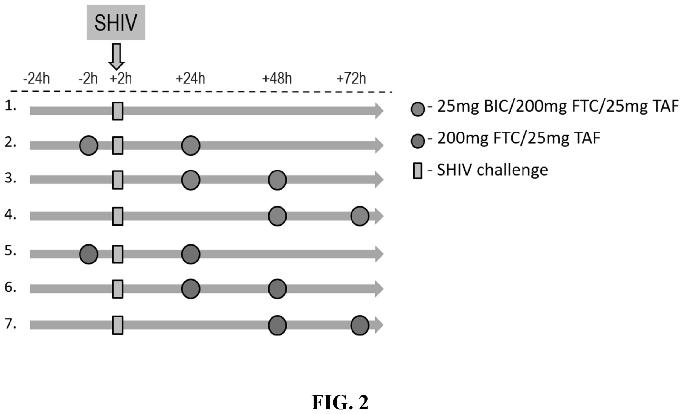

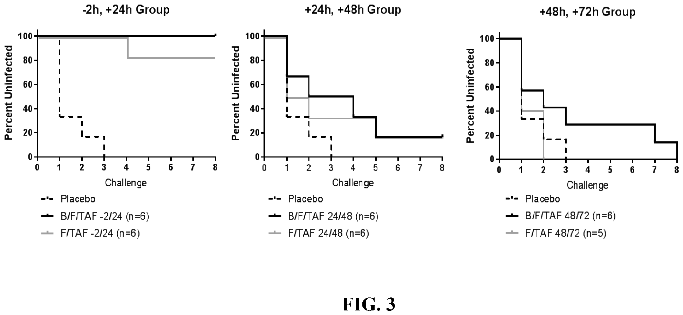

In a previous study, the efficacy of infection prevention was compared with two Antiretroviral Therapy (ART) regimens consisting of either a cocktail of 25 mg BIC (bictegravir), 200 mg FTC (emtricitabine), and 25 mg TAF (tenofovir alafenamide) or 200 mg FTC and 25 mg TAF in a repeat low-dose rectal challenge with SHIV162P3. The animals were stratified into three dosing regimens (groups 2-4 or 5-7), where the two of the above drug combinations were administered either before or after the exposure as summarized in FIG. 2. The rate of infection was established by measuring plasma viremia two weeks post challenge. Following eight challenge cycles, there was near complete protection afforded with -2hr/+24hr regimen (0/6 infected in BIC/FTC/TAF arm and 1/6 in FTC/TAF arm), minimal protection with +24hr/+48hr regimen (5/6 BIC/FTC/TAF arm and 5/6 FTC/TAF arm), and no protection afforded with +48hr/+72hr regimen (6/6 BIC/FTC/TAF arm and 5/5 FTC/TAF arm). Compared to the placebo control group, where all 6 animals became infected within three challenges, significant protection was observed only in the second and fifth groups, where treatment was initiated sooner than 24 hours post-exposure (FIG. 3).

In view of the results described above, a study was performed to determine whether an increase in BIC dose to 100 mg can improve the rate of protection when treatment is initiated 24 hours post-exposure or later. The second objective was to determine whether post-exposure treatment initiation could be protective if initiated earlier than 24 hours post-exposure with either a triple or double-drug regimen (*e*.*g*. 6 or 12 hours after the exposure).

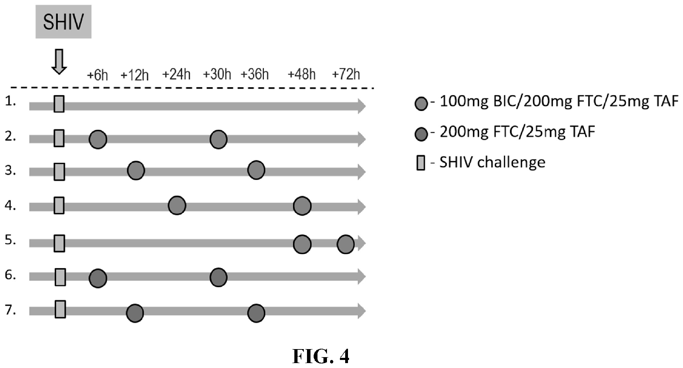

The following dosing regimens were tested (shown schematically in FIG. 4):
1. Placebo control
2. 100mg BIC, 25mg TAF, 200mg FTC dosed at +6, +30 post SHIV challenge
3. 100mg BIC, 25mg TAF, 200mg FTC dosed at +12, +36 post SHIV challenge
4. 100mg BIC, 25mg TAF, 200mg FTC dosed at +24, +48 post SHIV challenge
5. 100mg BIC, 25mg TAF, 200mg FTC dosed at +48, +72 post SHIV challenge
6. 25mg TAF, 200mg FTC dosed at +6, +30 post SHIV challenge
7. 25mg TAF, 200mg FTC dosed at +12, +36 post SHIV challenge

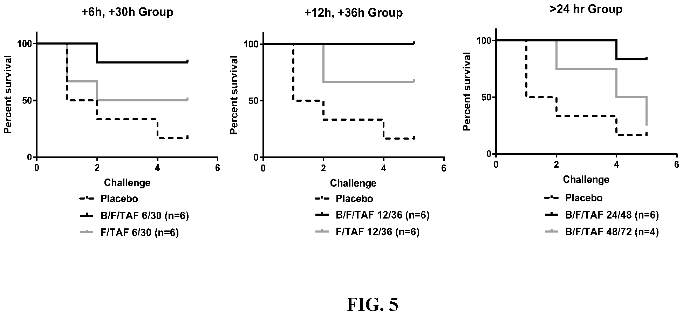

The rate of infection after five challenges (intrarectal route with SHIV162P3) in each of the seven test groups is shown in FIG. 5 and in Table 4, below.

**Table 4. Fraction SHIV+ total**

| | **Group** | **Challenge 1** | **Challenge 2** | **Challenge 3** | **Challenge 4** | **Challenge 5** | **P-value* vs Placebo** |
|---|---|---|---|---|---|---|---|
| **1** | Placebo | 3/6 | 4/6 | 4/6 | 5/6 | 5/6 | |
| **2** | B/F/TAF 6/30 | 0/6 | 1/6 | 1/6 | 1/6 | 1/6 | 0.0204 |
| **3** | B/F/TAF 12/36 | 0/6 | 0/6 | 0/6 | 0/6 | 0/6 | 0.0039 |
| **4** | B/F/TAF 24/48 | 0/6 | 0/6 | 0/6 | 1/6 | 1/6 | 0.0132 |
| **5** | B/F/TAF 48/72 | 0/6 | 1/6 | 1/6 | 2/6 | 3/4 | 0.3314 |
| **6** | F/TAF 6/30 | 2/6 | 3/6 | 3/6 | 3/6 | 3/6 | 0.3009 |
| **7** | F/TAF 12/36 | 0/6 | 2/6 | 2/6 | 2/6 | 2/6 | 0.0608 |

Significant protection was observed in groups 2, 3, and 4 (100mg BIC, 25mg TAF, 200mg FTC dosed at +6, +30 post SHIV challenge; +12, +36 post SHIV challenge; and +24, +48 post SHIV challenge).

It should be appreciated that certain features of the invention, which are, for clarity, described in the context of separate embodiments, can also be provided in combination in a single embodiment. Conversely, various features of the invention which are, for brevity, described in the context of a single embodiment, can also be provided separately or in any suitable subcombination.

All references, including publications, patents, and patent documents are incorporated by reference herein, as though individually incorporated by reference. The present disclosure provides reference to various embodiments and techniques. However, it should be understood that many variations and modifications may be made while remaining within the spirit and scope of the present disclosure.

The present disclosure provides the following clauses:
1. A method of preventing an HIV infection in a subject, comprising administering to the subject bictegravir, or a pharmaceutically acceptable salt thereof.
2. The method of clause 1, wherein the bictegravir, or a pharmaceutically acceptable salt thereof, is administered in a dosage of from about 10 mg/day to about 200 mg/day.
3. The method of clause 1, wherein the bictegravir, or a pharmaceutically acceptable salt thereof, is administered in a dosage of from about 10 mg/day to about 100 mg/day.
4. The method of clause 1, wherein the bictegravir, or a pharmaceutically acceptable salt thereof, is administered in a dosage of from about 25 mg/day to about 75 mg/day.
5. The method of clause 1 or 2, wherein the bictegravir, or a pharmaceutically acceptable salt thereof, is administered in a dosage of about 100 mg/day.
6. The method of clause 1, wherein the bictegravir, or a pharmaceutically acceptable salt thereof, is administered in a dosage of about 50 mg/day.
7. The method of any one of clauses 1 to 6, further comprising administering one to three additional therapeutic agents to the subject.
8. The method of clause 7, wherein the bictegravir, or a pharmaceutically acceptable salt thereof, and one to three additional therapeutic agents are administered simultaneously.
9. The method of clause 7 or 8, wherein the bictegravir, or a pharmaceutically acceptable salt thereof, and one to three additional therapeutic agents are administered as a unitary dosage form.
10. The method of any one of clauses 7 to 9, wherein the bictegravir, or a pharmaceutically acceptable salt thereof, and one to three additional therapeutic agents are administered as a fixed dose combination tablet.
11. The method of clause 7, wherein the bictegravir, or a pharmaceutically acceptable salt thereof, and one to three additional therapeutic agents are administered sequentially.
12. The method of any one of clauses 7 to 11, wherein each of the additional therapeutic agents is independently selected from an HIV protease inhibiting compound, an HIV non-nucleoside inhibitor of reverse transcriptase, an HIV nucleoside inhibitor of reverse transcriptase, an HIV nucleotide inhibitor of reverse transcriptase, an HIV integrase inhibitor, a gp41 inhibitor, a CXCR4 inhibitor, a gp120 inhibitor, a CCR5 inhibitor, and an HIV capsid inhibitor, or any combination thereof.
13. The method of any one of clauses 7 to 12, wherein one additional therapeutic agent is emtricitabine, or a pharmaceutically acceptable salt thereof.
14. The method of clause 13, wherein the emtricitabine, or a pharmaceutically acceptable salt thereof, is administered in a dosage of from about 100 mg/day to about 300 mg/day.
15. The method of clause 13, wherein the emtricitabine, or a pharmaceutically acceptable salt thereof, is administered in a dosage of from about 175 mg/day to about 225 mg/day.
16. The method of clause 13, wherein the emtricitabine, or a pharmaceutically acceptable salt thereof, is administered in a dosage of about 200 mg/day.
17. The method of any one of clauses 7 to 16, wherein one additional therapeutic agent is selected from tenofovir alafenamide, or a pharmaceutically acceptable salt thereof, and tenofovir disoproxil, or a pharmaceutically acceptable salt thereof.
18. The method of any one of clauses 7 to 17, wherein one additional therapeutic agent which is tenofovir alafenamide, or a pharmaceutically acceptable salt thereof.
19. The method of clause 17 or 18, wherein the tenofovir alafenamide, or a pharmaceutically acceptable salt thereof, is administered in a dosage of from about 10 mg/day to about 50 mg/day.
20. The method of clause 17 or 18, wherein the tenofovir alafenamide, or a pharmaceutically acceptable salt thereof, is administered in a dosage of from about 20 mg/day to about 30 mg/day.
21. The method of clause 17 or 18, wherein the tenofovir alafenamide, or a pharmaceutically acceptable salt thereof, is administered in a dosage of about 25 mg/day.
22. The method of any one of clauses 18 to 21, wherein one additional therapeutic agent is tenofovir alafenamide hemifumarate.
23. The method of any one of clauses 7 to 12, comprising administering a first additional therapeutic agent which is emtricitabine and a second additional therapeutic agent which is tenofovir alafenamide, or a pharmaceutically acceptable salt thereof.
24. The method of any one of clauses 7 to 12, comprising administering a first additional therapeutic agent which is emtricitabine and a second additional therapeutic agent which is tenofovir alafenamide hemifumarate.
25. The method of any one of clauses 1 to 6, wherein the bictegravir, or a pharmaceutically acceptable salt thereof, is administered as a monotherapy.
26. The method of any one of clauses 1 to 25, wherein the bictegravir, or a pharmaceutically acceptable salt thereof, is administered daily.
27. The method of any one of clauses 1 to 26, wherein the method comprises event driven administration of the bictegravir, or a pharmaceutically acceptable salt thereof, to the subject.
28. The method of clause 27, wherein the event driven administration comprises pre-exposure prophylaxis (PrEP).
29. The method of clause 27, wherein the event driven administration comprises post-exposure prophylaxis (PEP).
30. The method of clause 27, wherein the event driven administration comprises pre-exposure prophylaxis (PrEP) and post-exposure prophylaxis (PEP).
31. The method of any one of clauses 1 to 30, wherein the bictegravir, or a pharmaceutically acceptable salt thereof, is administered before exposure of the subject to the HIV.
32. The method of clause 31, wherein the bictegravir, or a pharmaceutically acceptable salt thereof, is administered as a single dose between 7 days and one day before exposure of the subject to the HIV.
33. The method of clause 31, wherein the bictegravir, or a pharmaceutically acceptable salt thereof, is administered from about 72 hours to about 1 hour before exposure of the subject to the HIV.
34. The method of clause 31, wherein the bictegravir, or a pharmaceutically acceptable salt thereof, is administered from about 24 hours to about 1 hour before exposure of the subject to the HIV.
35. The method of clause 31, wherein the bictegravir, or a pharmaceutically acceptable salt thereof, is administered from about 72 hours to about 24 hours before exposure of the subject to the HIV.
36. The method any one of clauses 1 to 28 and 30 to 35, wherein the pre-exposure prophylaxis (PrEP) comprises continuous PrEP.
37. The method of clause 36, wherein the continuous PrEP comprises daily administration of the bictegravir, or a pharmaceutically acceptable salt thereof, from about 7 days to about 2 hours before the exposure of the subject to the HIV.
38. The method of any one of clauses 1 to 37, comprising administering the bictegravir, or a pharmaceutically acceptable salt thereof, during the period of exposure of the subject to the HIV.
39. The method of clause 38, wherein the bictegravir, or a pharmaceutically acceptable salt thereof, is administered daily during the period of exposure of the subject to the HIV.
40. The method of clause 38, wherein the bictegravir, or a pharmaceutically acceptable salt thereof, is administered in a single dosage during the period of exposure of the subject to the HIV.
41. The method of any one of clauses 1 to 40, further comprising administering the bictegravir, or a pharmaceutically acceptable salt thereof, after exposure of the subject to the HIV.
42. The method of clause 41, wherein the bictegravir, or a pharmaceutically acceptable salt thereof, is administered from about 1 hour to about 72 hours after final exposure of the subject to the HIV.
43. The method of clause 41, wherein the bictegravir, or a pharmaceutically acceptable salt thereof, is administered from about 1 hour to about 24 hours after final exposure of the subject to the HIV.
44. The method of clause 41, wherein the bictegravir, or a pharmaceutically acceptable salt thereof, is administered from about 24 hours to about 72 hours after final exposure of the subject to the HIV.
45. The method of clause 1, wherein the method comprises:
   i) administering the bictegravir, or a pharmaceutically acceptable salt thereof, at least 7 days prior to exposure of the subject to the HIV;
   ii) administration of the bictegravir, or a pharmaceutically acceptable salt, daily during the period of exposure to the HIV; and
   iii) administration of the bictegravir, or a pharmaceutically acceptable salt thereof, within about 24 hours after the last exposure of the subject to the HIV.
46. The method of clause 1, wherein the method comprises:
   i) daily administration of the bictegravir, or a pharmaceutically acceptable salt thereof, beginning at least 7 days prior to exposure of the subject to the HIV;
   ii) daily administration of the bictegravir, or a pharmaceutically acceptable salt, during the period of exposure to the HIV; and
   iii) administration of the bictegravir, or a pharmaceutically acceptable salt thereof, within about 24 hours after the last exposure of the subject to the HIV.
47. The method of clause 1, wherein the method comprises:
   i) administering the bictegravir, or a pharmaceutically acceptable salt thereof, within 24 to 2 hours prior to exposure of the subject to the HIV;
   ii) administration of the bictegravir, or a pharmaceutically acceptable salt, daily during the period of exposure to the HIV; and
   iii) administration of the bictegravir, or a pharmaceutically acceptable salt thereof, within about 24 hours after the last exposure of the subject to the HIV.
48. The method of clause 47, further comprising a final administration of the bictegravir, or a pharmaceutically acceptable salt thereof, about 24 hours after the last exposure of the subject to the HIV.
49. The method of clause 1, wherein the method comprises:
   i) administering the bictegravir, or a pharmaceutically acceptable salt thereof, within 24 hours prior to exposure of the subject to the HIV;
   ii) administration of the bictegravir, or a pharmaceutically acceptable salt, in a single dosage during the period of exposure to the HIV; and
   iii) administration of the bictegravir, or a pharmaceutically acceptable salt thereof, in a single dosage within about 24 hours after the last exposure of the subject to the HIV.
50. The method of clause 1, wherein the method comprises:
   i) a first administration of the bictegravir, or a pharmaceutically acceptable salt thereof, within or at about 24 hours after exposure of the subject to the HIV; and
   ii) a second administration of the bictegravir, or a pharmaceutically acceptable salt thereof, within or at about 24 hours after the first administration.
51. The method of clause 1, wherein the method comprises:
   i) a first administration of the bictegravir, or a pharmaceutically acceptable salt thereof, at about 6 hours after exposure of the subject to the HIV; and
   ii) a second administration of the bictegravir, or a pharmaceutically acceptable salt thereof, at about 30 hours after exposure of the subject to the HIV.
52. The method of clause 1, wherein the method comprises:
   i) a first administration of the bictegravir, or a pharmaceutically acceptable salt thereof, at about 12 hours after exposure of the subject to the HIV; and
   ii) a second administration of the bictegravir, or a pharmaceutically acceptable salt thereof, at about 36 hours after exposure of the subject to the HIV.
53. The method of clause 1, wherein the method comprises:
   i) a first administration of the bictegravir, or a pharmaceutically acceptable salt thereof, at about 24 hours after exposure of the subject to the HIV; and
   ii) a second administration of the bictegravir, or a pharmaceutically acceptable salt thereof, at about 48 hours after exposure of the subject to the HIV.
54. The method of any one of clauses 50-53, wherein the bictegravir, or a pharmaceutically acceptable salt thereof, is administered in a dosage of about 100 mg at the first and second administrations.
55. The method of any one of clauses 50-53, wherein the bictegravir, or a pharmaceutically acceptable salt thereof, is administered in a dosage of about 50 mg at the first and second administrations.
56. The method of any one of clauses 50-55, wherein each of the first and second administrations of bictegravir, or a pharmaceutically acceptable salt thereof, further comprise administration of emtricitabine, or a pharmaceutically acceptable salt thereof, and tenofovir alafenamide, or a pharmaceutically acceptable salt thereof.
57. The method of any one of clauses 50-55, wherein each of the first and second administrations of bictegravir, or a pharmaceutically acceptable salt thereof, further comprise administration of emtricitabine, or a pharmaceutically acceptable salt thereof, in a dosage of about 200 mg and tenofovir alafenamide, or a pharmaceutically acceptable salt thereof, in a dosage of about 25 mg.
58. The method of any one of clauses 50-55, wherein each of the first and second administrations of bictegravir, or a pharmaceutically acceptable salt thereof, further comprise administration of emtricitabine, or a pharmaceutically acceptable salt thereof, in a dosage of about 400 mg and tenofovir alafenamide, or a pharmaceutically acceptable salt thereof, in a dosage of about 50 mg.
59. The method of any one of clauses 1 to 58, wherein the bictegravir, or a pharmaceutically acceptable salt thereof, is administered orally.
60. The method of any one of clauses 1 to 58, wherein the bictegravir, or a pharmaceutically acceptable salt thereof, is administered parenterally.
61. The method of clause 60, wherein the parenteral administration is selected from sub-cutaneous administration, intramuscular administration, transdermal administration, and vaginal administration.
62. The method of any one of clauses 1 to 61, wherein the bictegravir, or a pharmaceutically acceptable salt thereof, is administered to the subject through a medical device.
63. The method of clause 62, wherein the medical device is selected from a patch, an implantable device, a syringe, and a contraceptive device.
64. The method of any one of clauses 1 to 63, wherein the bictegravir is administered as bictegravir sodium.
65. A method of preventing an HIV infection in a subject, comprising administering to the subject bictegravir sodium in a dosage of from about 10 mg/day to about 100 mg/day;
   wherein the administration is performed at least 7 days prior to exposure of the subject to the HIV; administration is continued daily during the period of exposure to the HIV; administration is performed within 24 hours after the last exposure of the subject to the HIV; and a final administration is performed about 24 hours after the last exposure of the subject to the HIV.
66. A method of preventing an HIV infection in a subject, comprising administering to the subject bictegravir sodium in a dosage of from about 10 mg/day to about 100 mg/day, wherein the administration is performed about 72 hours to about 1 hour before exposure of the subject to the HIV or about 1 hour to about 72 hours after exposure of the subject to the HIV.
67. The method of clause 65, further comprising daily administration of the bictegravir sodium during the period of exposure of the subject to the HIV.
68. A method of preventing an HIV infection in a subject, comprising administering to the subject:
   (a) bictegravir sodium, in a dosage of about 10 mg/day to about 200 mg/day;
   (b) emtricitabine in a dosage of about 100 mg/day to about 400 mg/day; and
   (c) tenofovir alafenamide hemifumarate in a dosage of about 10 mg/day to about 50 mg/day;
   wherein the administration is performed one to three times about 1 hour to about 72 hours after exposure of the subject to the HIV.
69. A method of preventing an HIV infection in a subject, comprising administering to the subject:
   (a) bictegravir sodium in a dosage of from about 10 mg/day to about 100 mg/day;
   (b) emtricitabine in a dosage of from about 100 mg/day to about 300 mg/day; and
   (c) tenofovir alafenamide hemifumarate in a dosage of from about 10 mg/day to about 50 mg/day;
   wherein the administration is performed at least 7 days prior to exposure of the subject to the HIV; administration is continued daily during the period of exposure to the HIV; administration is performed within about 24 hours after the last exposure of the subject to the HIV; and a final administration is performed about 24 hours after the last exposure of the subject to the HIV.
70. A method of preventing an HIV infection in a subject, comprising administering to the subject:
   (a) bictegravir sodium in a dosage of from about 10 mg/day to about 100 mg/day;
   (b) emtricitabine in a dosage of from about 100 mg/day to about 300 mg/day; and
   (c) tenofovir alafenamide hemifumarate in a dosage of from about 10 mg/day to about 50 mg/day;
   wherein the administration is performed about 72 hours to about 1 hour before exposure of the subject to the HIV or about 1 hour to about 72 hours after exposure of the subject to the HIV.
71. The method of clause 70, further comprising daily administration of the bictegravir sodium, emtricitabine, and tenofovir alafenamide hemifumarate, during the period of exposure of the subject to the HIV.
72. A method of reducing the risk of acquiring HIV in a subject, comprising administering to the subject bictegravir sodium in a dosage of from about 10 mg/day to about 100 mg/day, wherein the administration is performed about 72 hours to about 1 hour before exposure of the subject to the HIV or about 1 hour to about 72 hours after exposure of the subject to the HIV.
73. The method of clause 72, further comprising daily administration of the bictegravir sodium during the period of exposure of the subject to the HIV.
74. The method of clause 72 or 73, wherein the reduction in risk of acquiring HIV is at least about 75% compared to a subject having not been administered the bictegravir sodium.
75. A method of reducing the risk of acquiring HIV in a subject, comprising administering to the subject:
   (a) bictegravir sodium, in a dosage of about 10 mg/day to about 200 mg/day;
   (b) emtricitabine in a dosage of about 100 mg/day to about 400 mg/day; and
   (c) tenofovir alafenamide hemifumarate in a dosage of about 10 mg/day to about 50 mg/day;
   wherein the administration is performed one to three times about 1 hour to about 72 hours after exposure of the subject to the HIV.
76. A method of reducing the risk of acquiring HIV in a subject, comprising administering to the subject:
   (a) bictegravir sodium in a dosage of from about 10 mg/day to about 100 mg/day;
   (b) emtricitabine in a dosage of from about 100 mg/day to about 300 mg/day; and
   (c) tenofovir alafenamide hemifumarate in a dosage of from about 10 mg/day to about 50 mg/day;
   wherein the administration is performed about 72 hours to about 1 hour before exposure of the subject to the HIV or about 1 hour to about 72 hours after exposure of the subject to the HIV.
77. The method of clause 76, further comprising daily administration of the bictegravir sodium, emtricitabine, and tenofovir alafenamide hemifumarate, during the period of exposure of the subject to the HIV.
78. The method of clause 76 or 77, wherein the reduction in risk of acquiring HIV is at least about 75% compared to a subject having not been administered the bictegravir sodium, emtricitabine, and tenofovir alafenamide hemifumarate.
79. The method of any one of clauses 1 to 78, wherein the subject has been identified as an individual who is at risk of sexual transmission of HIV.
80. The method of any one of clauses 1 to 79, wherein the HIV is HIV-1.
81. The method of any one of clauses 1 to 79, wherein the HIV is HIV-2.

## Claims

1. A method of preventing an HIV infection in a subject, comprising administering to the subject bictegravir, or a pharmaceutically acceptable salt thereof.

2. The method of claim 1, wherein the bictegravir, or a pharmaceutically acceptable salt thereof, is administered in a dosage of:
(i) from about 10 mg/day to about 200 mg/day;
(ii) from about 10 mg/day to about 100 mg/day;
(iii) about 25 mg/day to about 75 mg/day;
(iv) about 100 mg/day; or
(v) about 50 mg/day.

3. The method of claim 1 or 2, further comprising administering one to three additional therapeutic agents to the subject, optionally wherein:
(i) the bictegravir, or a pharmaceutically acceptable salt thereof, and one to three additional therapeutic agents are administered:
(a) simultaneously;
(b) as a unitary dosage form; and/or
(c) as a fixed dose combination tablet, or
(ii) the bictegravir, or a pharmaceutically acceptable salt thereof, and one to three additional therapeutic agents are administered sequentially.

4. The method of claim 3, wherein each of the additional therapeutic agents is independently selected from an HIV protease inhibiting compound, an HIV non-nucleoside inhibitor of reverse transcriptase, an HIV nucleoside inhibitor of reverse transcriptase, an HIV nucleotide inhibitor of reverse transcriptase, an HIV integrase inhibitor, a gp41 inhibitor, a CXCR4 inhibitor, a gp120 inhibitor, a CCR5 inhibitor, and an HIV capsid inhibitor, or any combination thereof.

5. The method of claim 3 or 4, wherein one additional therapeutic agent is:
(i) emtricitabine, or a pharmaceutically acceptable salt thereof, optionally wherein the emtricitabine, or a pharmaceutically acceptable salt thereof, is administered in a dosage of:
(a) from about 100 mg/day to about 300 mg/day;
(b) from about 175 mg/day to about 225 mg/day; or
(c) about 200 mg/day;
(ii) selected from tenofovir alafenamide, or a pharmaceutically acceptable salt thereof, and tenofovir disoproxil, or a pharmaceutically acceptable salt thereof; and/or
(iii) tenofovir alafenamide, or a pharmaceutically acceptable salt thereof, optionally wherein the tenofovir alafenamide, or a pharmaceutically acceptable salt thereof, is administered in a dosage of:
(a) from about 10 mg/day to about 50 mg/day;
(b) from about 20 mg/day to about 30 mg/day; or
(c) about 25 mg/day.

6. The method of claim 5(iii), wherein one additional therapeutic agent is tenofovir alafenamide hemifumarate.

7. The method of claim 3 or 4, comprising administering a first additional therapeutic agent which is emtricitabine and a second additional therapeutic agent which is:
(i) tenofovir alafenamide, or a pharmaceutically acceptable salt thereof; or
(ii) tenofovir alafenamide hemifumarate.

8. The method of claim 1 or 2, wherein the bictegravir, or a pharmaceutically acceptable salt thereof, is administered as a monotherapy.

9. The method of any one of claims 1 to 8, wherein the bictegravir, or a pharmaceutically acceptable salt thereof, is administered daily, and/or wherein
the method comprises event driven administration of the bictegravir, or a pharmaceutically acceptable salt thereof, to the subject, optionally wherein the event driven administration comprises:
(i) pre-exposure prophylaxis (PrEP);
(ii) post-exposure prophylaxis (PEP); or
(iii) pre-exposure prophylaxis (PrEP) and post-exposure prophylaxis (PEP).

10. The method of any one of claims 1 to 9, wherein the bictegravir, or a pharmaceutically acceptable salt thereof, is administered before exposure of the subject to the HIV, optionally wherein the bictegravir, or a pharmaceutically acceptable salt thereof, is administered:
(i) as a single dose between 7 days and one day before exposure of the subject to the HIV;
(ii) from about 72 hours to about 1 hour before exposure of the subject to the HIV;
(iii) from about 24 hours to about 1 hour before exposure of the subject to the HIV; or
(iv) from about 72 hours to about 24 hours before exposure of the subject to the HIV.

11. The method any one of claims 1 to 10, wherein the pre-exposure prophylaxis (PrEP) comprises continuous PrEP, optionally wherein the continuous PrEP comprises daily administration of the bictegravir, or a pharmaceutically acceptable salt thereof, from about 7 days to about 2 hours before the exposure of the subject to the HIV.

12. The method of any one of claims 1 to 11, comprising administering the bictegravir, or a pharmaceutically acceptable salt thereof, during the period of exposure of the subject to the HIV, optionally wherein the bictegravir, or a pharmaceutically acceptable salt thereof, is administered:
(i) daily during the period of exposure of the subject to the HIV; or
(ii) in a single dosage during the period of exposure of the subject to the HIV, and/or wherein
the method further comprises administering the bictegravir, or a pharmaceutically acceptable salt thereof, after exposure of the subject to the HIV, optionally wherein
the bictegravir, or a pharmaceutically acceptable salt thereof, is administered from:
(i) about 1 hour to about 72 hours after final exposure of the subject to the HIV;
(ii) about 1 hour to about 24 hours after final exposure of the subject to the HIV; or
(iii) about 24 hours to about 72 hours after final exposure of the subject to the HIV.

13. The method of claim 1, wherein:
(A) the method comprises:
i) administering the bictegravir, or a pharmaceutically acceptable salt thereof, at least 7 days prior to exposure of the subject to the HIV;
ii) administration of the bictegravir, or a pharmaceutically acceptable salt, daily during the period of exposure to the HIV; and
iii) administration of the bictegravir, or a pharmaceutically acceptable salt thereof, within about 24 hours after the last exposure of the subject to the HIV;
(B) the method comprises:
i) daily administration of the bictegravir, or a pharmaceutically acceptable salt thereof, beginning at least 7 days prior to exposure of the subject to the HIV;
ii) daily administration of the bictegravir, or a pharmaceutically acceptable salt, during the period of exposure to the HIV; and
iii) administration of the bictegravir, or a pharmaceutically acceptable salt thereof, within about 24 hours after the last exposure of the subject to the HIV;
(C) the method comprises:
i) administering the bictegravir, or a pharmaceutically acceptable salt thereof, within 24 to 2 hours prior to exposure of the subject to the HIV;
ii) administration of the bictegravir, or a pharmaceutically acceptable salt, daily during the period of exposure to the HIV; and
iii) administration of the bictegravir, or a pharmaceutically acceptable salt thereof, within about 24 hours after the last exposure of the subject to the HIV, optionally wherein the method further comprises a final administration of the bictegravir, or a pharmaceutically acceptable salt thereof, about 24 hours after the last exposure of the subject to the HIV;
(D) the method comprises:
i) administering the bictegravir, or a pharmaceutically acceptable salt thereof, within 24 hours prior to exposure of the subject to the HIV;
ii) administration of the bictegravir, or a pharmaceutically acceptable salt, in a single dosage during the period of exposure to the HIV; and
iii) administration of the bictegravir, or a pharmaceutically acceptable salt thereof, in a single dosage within about 24 hours after the last exposure of the subject to the HIV;
(E) the method comprises:
i) a first administration of the bictegravir, or a pharmaceutically acceptable salt thereof, within or at about 24 hours after exposure of the subject to the HIV; and
ii) a second administration of the bictegravir, or a pharmaceutically acceptable salt thereof, within or at about 24 hours after the first administration, optionally wherein
the bictegravir, or a pharmaceutically acceptable salt thereof, is administered in a dosage of:
(i) about 100 mg at the first and second administrations; or
(ii) about 50 mg at the first and second administrations;
(F) the method comprises:
i) a first administration of the bictegravir, or a pharmaceutically acceptable salt thereof, at about 6 hours after exposure of the subject to the HIV; and
ii) a second administration of the bictegravir, or a pharmaceutically acceptable salt thereof, at about 30 hours after exposure of the subject to the HIV, optionally wherein
the bictegravir, or a pharmaceutically acceptable salt thereof, is administered in a dosage of:
(i) about 100 mg at the first and second administrations; or
(ii) about 50 mg at the first and second administrations;
(G) the method comprises:
i) a first administration of the bictegravir, or a pharmaceutically acceptable salt thereof, at about 12 hours after exposure of the subject to the HIV; and
ii) a second administration of the bictegravir, or a pharmaceutically acceptable salt thereof, at about 36 hours after exposure of the subject to the HIV, optionally wherein
the bictegravir, or a pharmaceutically acceptable salt thereof, is administered in a dosage of:
(i) about 100 mg at the first and second administrations; or
(ii) about 50 mg at the first and second administrations; or
(H) the method comprises:
i) a first administration of the bictegravir, or a pharmaceutically acceptable salt thereof, at about 24 hours after exposure of the subject to the HIV; and
ii) a second administration of the bictegravir, or a pharmaceutically acceptable salt thereof, at about 48 hours after exposure of the subject to the HIV; optionally wherein
the bictegravir, or a pharmaceutically acceptable salt thereof, is administered in a dosage of:
(i) about 100 mg at the first and second administrations; or
(ii) about 50 mg at the first and second administrations.

14. The method of any one of claim 13(E)-(H), wherein
each of the first and second administrations of bictegravir, or a pharmaceutically acceptable salt thereof, further comprise administration of:
(i) emtricitabine, or a pharmaceutically acceptable salt thereof, and tenofovir alafenamide, or a pharmaceutically acceptable salt thereof;
(ii) emtricitabine, or a pharmaceutically acceptable salt thereof, in a dosage of about 200 mg and tenofovir alafenamide, or a pharmaceutically acceptable salt thereof, in a dosage of about 25 mg; or
(iii) emtricitabine, or a pharmaceutically acceptable salt thereof, in a dosage of about 400 mg and tenofovir alafenamide, or a pharmaceutically acceptable salt thereof, in a dosage of about 50 mg.

15. The method of any one of claims 1 to 14, wherein:
(i) the bictegravir, or a pharmaceutically acceptable salt thereof, is administered:
(a) orally; or
(b) parenterally, optionally wherein
the parenteral administration is selected from sub-cutaneous administration, intramuscular administration, transdermal administration, and vaginal administration;
(ii) the bictegravir, or a pharmaceutically acceptable salt thereof, is administered to the subject through a medical device, optionally wherein
the medical device is selected from a patch, an implantable device, a syringe, and a contraceptive device; and/or
(iii) the bictegravir is administered as bictegravir sodium.

16. A method of preventing an HIV infection in a subject, comprising administering to the subject:
(A) bictegravir sodium in a dosage of from about 10 mg/day to about 100 mg/day; wherein
the administration is performed at least 7 days prior to exposure of the subject to the HIV; administration is continued daily during the period of exposure to the HIV; administration is performed within 24 hours after the last exposure of the subject to the HIV; and a final administration is performed about 24 hours after the last exposure of the subject to the HIV;
(B) bictegravir sodium in a dosage of from about 10 mg/day to about 100 mg/day, wherein
the administration is performed about 72 hours to about 1 hour before exposure of the subject to the HIV or about 1 hour to about 72 hours after exposure of the subject to the HIV, optionally wherein
the method further comprises daily administration of the bictegravir sodium during the period of exposure of the subject to the HIV;
(C)
(a) bictegravir sodium, in a dosage of about 10 mg/day to about 200 mg/day;
(b) emtricitabine in a dosage of about 100 mg/day to about 400 mg/day; and
(c) tenofovir alafenamide hemifumarate in a dosage of about 10 mg/day to about 50 mg/day; wherein
the administration is performed one to three times about 1 hour to about 72 hours after exposure of the subject to the HIV;
(D)
(a) bictegravir sodium in a dosage of from about 10 mg/day to about 100 mg/day;
(b) emtricitabine in a dosage of from about 100 mg/day to about 300 mg/day; and
(c) tenofovir alafenamide hemifumarate in a dosage of from about 10 mg/day to about 50 mg/day; wherein
the administration is performed at least 7 days prior to exposure of the subject to the HIV; administration is continued daily during the period of exposure to the HIV; administration is performed within about 24 hours after the last exposure of the subject to the HIV; and a final administration is performed about 24 hours after the last exposure of the subject to the HIV; or
(E)
(a) bictegravir sodium in a dosage of from about 10 mg/day to about 100 mg/day;
(b) emtricitabine in a dosage of from about 100 mg/day to about 300 mg/day; and
(c) tenofovir alafenamide hemifumarate in a dosage of from about 10 mg/day to about 50 mg/day; wherein
the administration is performed about 72 hours to about 1 hour before exposure of the subject to the HIV or about 1 hour to about 72 hours after exposure of the subject to the HIV, optionally wherein the method further comprises daily administration of the bictegravir sodium, emtricitabine, and tenofovir alafenamide hemifumarate, during the period of exposure of the subject to the HIV.

17. A method of reducing the risk of acquiring HIV in a subject, comprising administering to the subject:
(A) bictegravir sodium in a dosage of from about 10 mg/day to about 100 mg/day, wherein the administration is performed about 72 hours to about 1 hour before exposure of the subject to the HIV or about 1 hour to about 72 hours after exposure of the subject to the HIV, optionally wherein
the method further comprises daily administration of the bictegravir sodium during the period of exposure of the subject to the HIV, and/or wherein
the reduction in risk of acquiring HIV is at least about 75% compared to a subject having not been administered the bictegravir sodium;
(B)
(a) bictegravir sodium, in a dosage of about 10 mg/day to about 200 mg/day;
(b) emtricitabine in a dosage of about 100 mg/day to about 400 mg/day; and
(c) tenofovir alafenamide hemifumarate in a dosage of about 10 mg/day to about 50 mg/day; wherein
the administration is performed one to three times about 1 hour to about 72 hours after exposure of the subject to the HIV; or
(C)
(a) bictegravir sodium in a dosage of from about 10 mg/day to about 100 mg/day;
(b) emtricitabine in a dosage of from about 100 mg/day to about 300 mg/day; and
(c) tenofovir alafenamide hemifumarate in a dosage of from about 10 mg/day to about 50 mg/day; wherein
the administration is performed about 72 hours to about 1 hour before exposure of the subject to the HIV or about 1 hour to about 72 hours after exposure of the subject to the HIV, optionally wherein
the method further comprises daily administration of the bictegravir sodium, emtricitabine, and tenofovir alafenamide hemifumarate, during the period of exposure of the subject to the HIV, and/or wherein
the reduction in risk of acquiring HIV is at least about 75% compared to a subject having not been administered the bictegravir sodium, emtricitabine, and tenofovir alafenamide hemifumarate.

18. The method of any one of claims 1 to 17, wherein the subject has been identified as an individual who is at risk of sexual transmission of HIV, optionally wherein the HIV is:
(i) HIV-1; or
(ii) HIV-2.
